# EUROPEAN PATENT SPECIFICATION

(11) **EP 3 216 796 B1**
(45) Date of publication and mention of the grant of the patent: **19.08.2020**
(21) Application number: 15857241.2
(22) Date of filing: 30.03.2015
(51) Int. Cl.: C07F 9/54, C07F 9/50, A61K 51/00, C07B 59/00, A61K 51/04

(54) **PHOSPHONIUM COMPOUND AND PRODUCTION METHOD THEREFOR**
PHOSPHONIUMVERBINDUNG UND HERSTELLUNGSVERFAHREN DAFÜR
COMPOSÉ DE PHOSPHONIUM ET SON PROCÉDÉ DE PRODUCTION

(30) Priority: 05.11.2014 JP 2014224986
(43) Date of publication of application: 13.09.2017
(73) Proprietor: Tohoku University, Sendai-shi, Miyagi 980-8577 (JP)
(72) Inventor: FURUMOTO, Shozo, Sendai-shi Miyagi 980-8577 (JP)
(74) Representative: Mewburn Ellis LLP
(86) International application number: PCT/JP2015/059857
(87) International publication number: WO 2016/072104

(56) References cited:
- EP-A1- 0 455 287
- WO-A1-2006/121035
- WO-A1-2013/172979
- CN-A- 102 898 470
- JP-A- H09 249 677
- JP-A- 2005 532 262
- JP-A- 2013 523 702
- DONG-YEON KIM ET AL: "Synthesis of [18F]-labeled (2-(2-fluoroethoxy)ethyl)tris(4-methoxyphe nyl)phosphonium cation as a potential agent for positron emission tomography myocardial imaging", NUCLEAR MEDICINE AND BIOLOGY., vol. 39, no. 7, 1 October 2012 (2012-10-01), pages 1093-1098, XP55469410, US ISSN: 0969-8051, DOI: 10.1016/j.nucmedbio.2012.03.008

## Description

### TECHNICAL FIELD

The present invention relates to a method for producing a quaternary phosphonium compound labeled with a radionuclide, in particular a positron emitting nuclide such as ¹⁸F. This invention also relates to a quaternary phosphonium compound labeled with a radionuclide, in particular a positron emitting nuclide such as ¹⁸F, and to an imaging agent comprising said compound.

### BACKGROUND ART

There is formed a proton concentration gradient across the inner mitochondrial membrane, and due to the concentration gradient, a membrane potential from -140 mV to -180 mV is generated in the inner mitochondrial membrane. Delocalized lipophilic cations (DLCs) are molecules having a structure that permits intramolecular delocalization of positive charge by resonance stabilization. DLCs have sufficient lipophilicity to easily pass through the lipid bilayer of a cell membrane, and have the characteristic of accumulating in mitochondria of living organisms utilizing a mitochondrial membrane electrochemical potential (Non-Patent Literatures 1 and 2).

Research has been made on the usage of radionuclide-labeled DLCs in various applications including myocardial perfusion imaging, apoptosis imaging, and imaging of brown adipose tissue (BAT) activity. In particular, there have been many reports concerning myocardial perfusion imaging.

At present, ischemic heart disease is the most common cause of death in the world. According to the survey made by the World Health Organization, seven million people died of this disease in 2011. In Japan, this disease ranks the second leading cause of death behind malignant neoplasm. Ischemic heart disease is a generic name for *angina pectoris,* heart infarction, and the like, and is mainly caused by the progression of arteriosclerosis of the coronary artery. For the purpose of effective treatment, early detection of myocardial ischemia is essential. Therefore, the development of a highly accurate diagnostic method which leads to early detection of ischemic heart disease may well be said to be one of the important challenges in modern medicine.

Examples of common detection methods for ischemic heart disease include not only electrocardiographic examination, ultrasonographic examination, blood examination, and cardiac catheter examination, but also myocardial perfusion imaging using a nuclear medicine examination method such as PET or SPECT. In patients with mild myocardial ischemia, the blood flow over the whole myocardium is maintained during rest, but when a stress is imposed on the myocardium due to exercise, etc., the myocardium requires large amounts of oxygen and nutrients and thus circulatory blood flow increases. While the blood flow increases in normal sites, coronary blood flow does not increase in arterioles distal to the site of narrowing, since blood vessels are dilated almost to the maximum during rest to maintain the blood flow. Thus, when imaging is performed under myocardial stress, the site of narrowing appears as an image of loss of radioactivity. Based on this principle, diagnosis of myocardial blood flow is done by imposing a stress on the myocardium of patients through exercise or the use of a medication like adenosine and comparing images taken during rest and stress.

In clinical settings, SPECT agents -- *e.g.,* [²⁰¹Tl]TlCl, and ^{99m}Tc-labeled formulations like [^{99m}Tc]sestamibi (MIBI) and [^{99m}Tc]tetrofosmin -- are now mainly used as myocardial perfusion imaging agents. However, SPECT has some limitations: for example, since there is no standardized radiation attenuation correction method available, the influence of radiation attenuation occurs in stout patients or large-breasted female patients; and also this method is unfit for quantitative analysis. So, there is an expectation to use a PET method characterized by high spatial resolution and sensitivity.

Apoptosis is a programmed form of cell death induced by the activation of a specific protease (caspase) present in cells. It is reported that the activation of caspase is mediated by cytochrome c released from mitochondria, and that the release of cytochrome c takes place in association with change in mitochondrial membrane potential. Mitochondria play an important role in the control of apoptosis (Non-Patent Literature 3).

Unlike white adipose responsible for energy storage, brown adipose tissue produces heat through energy consumption, and thus is one of the targets for the treatment of obesity. Brown adipose produces heat for body-temperature maintenance in cold environments or through consumption of high fat foods, and this heat production takes place in mitochondria abundant in brown adipose cells.

PET agents reported include [¹⁵O]H₂O, [¹³N]NH₃, and [⁸²Rb]RbCl. However, since ¹⁵O and ¹³N have a short half-life of 2 and 10 minutes, respectively, the use of [¹⁵O]H₂O and [¹³N]NH₃ is limited to clinical facilities equipped with a cyclotron. Further, ⁸²Rb is produced from ⁸²Sr in a generator, but since the generator is expensive, there is a limitation on the use of the generator for high-throughput applications. For these reasons, active research and development have in recent years been conducted on ¹⁸F-labeled PET imaging agents having a longer half-life (110 minutes) than the aforementioned nuclides (Patent Literatures 1 to 3, and Non-Patent Literatures 5 to 12). Non-Patent Literature 12 discusses ¹⁸F labelled phosphonium compounds.

### CITATION LIST

### PATENT LITERATURES

Patent Literature 1: Japanese Patent Domestic Publication No. JP 2005-532262
Patent Literature 2: Japanese Patent Application Publication No. JP 2012-52196
Patent Literature 3: Japanese Patent Domestic Publication No. JP 2013-523702

### NON-PATENT LITERATURES

Non-Patent Literature 1: Ross, M. F., et al., Biochemistry (Moscow) 2005, 70, 222;
Non-Patent Literature 2: Yousif, L. F., et al., ChemBioChem 2009, 10, 1939;
Non-Patent Literature 3: Tatton, W. G., et al., Biochim. Biophys. Acta, 1999, 1410, 195;
Non-Patent Literature 4: Madar, I., et al., J. Nucl. Med. 2011, 52, 808;
Non-Patent Literature 5: Ravert, H. T., et al., J. Label. Compd. Radiopharm. 2004, 47, 469;
Non-Patent Literature 6: Madar, I., et al., J. Nucl. Med. 2006, 47, 1359;
Non-Patent Literature 7: Madar, I., et al., J. Nucl. Med. 2007, 48, 1021;
Non-Patent Literature 8: Higuchi, T., et al., J. Nucl. Med. 2011, 52, 965;
Non-Patent Literature 9: Shoup, T. M., et al., J. Mol. Imaging Biol. 2011, 13, 511;
Non-Patent Literature 10: Kim, D. Y., et al., J. Bioconjug. Chem. 2012, 23, 431;
Non-Patent Literature 11: Tominaga, et al., Abstracts of Special Programs and General Presentations at the 53rd Annual Scientific Meeting of the Japanese Society of Nuclear Medicine, 197*.*
Non-Patent Literature 12: Kim, et al., Nuclear Medicine and Biology, 2012, 1093 to 1098

### SUMMARY OF INVENTION

### TECHNICAL PROBLEM

In 2004, Ravert, *et al.* reported the synthesis of 4-[¹⁸F]fluorobenzyl-triphenylphosphonium bromide ([¹⁸F]FBnTP) (Non-Patent Literature 5). Such phosphonium compounds having three phenyl groups have characteristics as delocalized lipophilic cations (DLCs) which permit intramolecular delocalization of positive charge by resonance stabilization. Due to their lipophilicity, DLCs are capable of easily passing through the lipid bilayer of a cell membrane and preferentially accumulating in the inner mitochondrial membrane which has a very low membrane potential. There are reports on myocardial perfusion imaging performed with [¹⁸F]FBnTP, targeting mitochondria abundant in myocardial cells (Non-Patent Literatures 6 to 8).

However, since ¹⁸F has a half-life of 110 minutes, there is a need for a synthesis method that enables efficient and short-time production of a compound for use as a PET imaging agent. In the process of synthesis of [¹⁸F]FBnTP, the use of a highly corrosive brominating agent is needed at the step of synthesizing benzyl bromide by bromination -- this use has caused a problem with automatic synthesis using a synthesizer. Also, [¹⁸F]FBnTP has the property of accumulating not only in the myocardium but also in the liver adjacent to the myocardium to the same extent, and so there is concern that [¹⁸F]FBnTP has a negative effect on myocardial perfusion imaging.

Shoup, *et al.* also reported PET myocardial imaging using (4-[¹⁸F]fluorophenyl)triphenylphosphonium (FTPP) (Non-Patent Literature 9). However, the synthesis of FTPP has some problems in that a high temperature of 200°C is required for labeling with ¹⁸F, and the radiochemical yield of [¹⁸F]FTPP is as low as about 10%. Likewise, (6-[¹⁸F]fluorohexyl)triphenylphosphonium reported by Kim, *et al.* (Non-Patent Literature 10) is problematic in practical use because the temperature condition of 220°C is used for its synthesis.

An object of the present invention is to provide a production method that enables effective and short-time production of a quaternary phosphonium compound labeled with a radionuclide, in particular a positron emitting nuclide such as ¹⁸F. Another object of this invention is to provide a quaternary phosphonium compound labeled with a radionuclide, in particular a positron emitting nuclide such as ¹⁸F, and especially to provide a quaternary phosphonium compound that is suitable as a PET imaging agent useful for imaging of the myocardium and the like.

### SOLUTION TO PROBLEM

The present inventors have made intensive studies to achieve the aforementioned objects, and found a highly effective production method for a quaternary phosphonium compound labeled with a radionuclide. The inventors also found that the quaternary phosphonium compounds that can be produced by the aforementioned method have favorable characteristics as PET imaging agents. Thus, the inventors have completed the present invention. The disclosure of the present application includes the invention set forth below in (1) to (24).
(1) A phosphonium compound of formula (II): 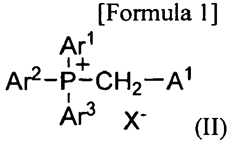
   wherein Ar¹, Ar², and Ar³ are each independently an aryl optionally substituted with one or more substituents selected from B², wherein at least one of Ar¹, Ar², and Ar³ is substituted with one or more substituents selected from radionuclide-labeled C₁₋₆alkyl, radionuclide-labeled C₂₋₆alkoxy, radionuclide-labeled C₂₋₆alkoxyC₁₋₆alkyl, radionuclide-labeled C₂₋₆alkoxyC₂₋₆alkoxy, radionuclide-labeled C₂₋₆alkoxyC₂₋₆alkoxyC₁₋₆alkyl, and radionuclide-labeled C₂₋₆alkoxyC₂₋₆alkoxyC₂₋₆alkoxy;
   A¹ is a hydrogen atom, C₁₋₁₀alkyl optionally substituted with one or more substituents selected from B¹, C₂₋₁₀alkenyl optionally substituted with one or more substituents selected from B¹, or an aryl optionally substituted with one or more substituents selected from B²;
   each B¹ is independently a halogen atom, C₁₋₆alkoxy, phenyl, or naphthyl, wherein the phenyl and naphthyl are optionally substituted with one or more substituents selected from C₁₋₆alkyl, C₁₋₆alkoxy, and halogen atoms;
   each B² is independently a halogen atom, C₁₋₆alkyl, C₁₋₆alkoxy, C₁₋₆alkylthio, cyano, amino, C₁₋₆alkylamino, di(C₁₋₆alkyl)amino, nitro, hydroxy, or (C₁₋₃alkoxy)carbonyl, wherein the alkyl, alkoxy and alkylthio are optionally substituted with one or more substituents selected from halogen atoms;
   Ar¹, Ar², Ar³ and A¹, and the substituents contained therein, optionally form acid addition salts;
   X⁻ is an anion with a total charge of -1.
(2) The phosphonium compound according to (1), wherein the radionuclide is a positron emitting nuclide.
(3) The phosphonium compound according to (2), wherein the positron emitting nuclide is ¹⁸F.
(4) The phosphonium compound according to any of (1) to (3), wherein A¹ is a hydrogen atom, C₁₋₆alkyl optionally substituted with one or more halogen atoms, phenyl, or phenylC₁₋₄alkyl, wherein the phenyl or a phenyl moiety of phenylC₁₋₄alkyl is optionally substituted with one or more substituents selected from one or more halogen atoms, C₁₋₆alkyl, C₁₋₆alkoxy, C₁₋₆alkylthio, cyano, amino, C₁₋₆alkylamino, di(C₁₋₆alkyl)amino, nitro, hydroxy, and (C₁₋₃alkoxy)carbonyl.
(5) The phosphonium compound according to (1), wherein the phosphonium compound comprises a phosphonium selected from:
   benzyl-[4-(2-[¹⁸F]fluoroethoxy)phenyl]-diphenylphosphonium;
   benzyl-(4-[2-{2-(2-[¹⁸F]fluoroethoxy)ethoxy} ethoxy]phenyl)-diphenylphosphonium;
   [4-(2-[¹⁸F]fluoroethoxy)phenyl]-(4-methoxycarbonylphenyl)methyl-diphenylphosphonium;
   [4-(2-[¹⁸F]fluoroethoxy)phenyl]-(4-fluorophenyl)methyl-diphenylphosphonium;
   [4-(2-[¹⁸F]fluoroethoxy)phenyl]-(3,4,5-trifluorophenyl)methyl-diphenylphosphonium;
   (4-chlorophenyl)methyl- [4-(2- [¹⁸F] fluoroethoxy)phenyl]-diphenylphosphonium;
   [4-(2-[¹⁸F]fluoroethoxy)phenyl]-(4-methoxyphenyl)methyl-diphenylphosphonium;
   [4-(2-[¹⁸F]fluoroethoxy)phenyl]-(n-pentyl)-diphenylphosphonium;
   [4-(2-[¹⁸F]fluoroethoxy)phenyl]-(3-phenylpropyl)-diphenylphosphonium;
   (4-n-butylphenyl)methyl-[4-(2-[¹⁸F]fluoroethoxy)phenyl]-diphenylphosphonium;
   (3-fluorophenyl)methyl-[4-(2-[¹⁸F]fluoroethoxy)phenyl]-diphenylphosphonium;
   [4-(2-[¹⁸F]fluoroethoxy)phenyl]-[4-(trifluoromethylthio)phenyl]methyl-diphenylphosphonium;
   [4-(2-[¹⁸F]fluoroethoxy)phenyl]-(2-methylphenyl)methyl-diphenylphosphonium;
   (3-cyanophenyl)methyl-[4-(2-[¹⁸F]fluoroethoxy)phenyl]-diphenylphosphonium;
   [4-(2-[¹⁸F]fluoroethoxy)phenyl]-methyl-diphenylphosphonium;
   allyl-[4-(2-[¹⁸F]fluoroethoxy)phenyl]-diphenylphosphonium;
   benzyl-[3-(2-[¹⁸F]fluoroethoxy)phenyl]-diphenylphosphonium;
   [3-(2-[¹⁸F]fluoroethoxy)phenyl]-(4-methoxyphenyl)methyl-diphenylphosphonium;
   [3-(2-[¹⁸F]fluoroethoxy)phenyl]-(4-methoxycarbonylphenyl)methyl-diphenylphosphonium;
   [3-(2-[¹⁸F]fluoroethoxy)phenyl]-(4-fluorophenyl)methyl-diphenylphosphonium;
   [3-(2-[¹⁸F]fluoroethoxy)phenyl]-(3-phenylpropyl)-diphenylphosphonium;
   (4-chlorophenyl)methyl-[3-(2-[¹⁸F]fluoroethoxy)phenyl]-diphenylphosphonium;
   [3 -(2- [¹⁸F] fluoroethoxy)phenyl]-(3,4,5-trifluorophenyl)methyl-diphenylphosphonium;
   benzyl-[3-(3-[¹⁸F]fluoropropoxy)phenyl]-diphenylphosphonium;
   benzyl-[3-(4-[¹⁸F]fluorobutoxy)phenyl]-diphenylphosphonium;
   [3-(2-[¹⁸F]fluoroethoxy)phenyl]-methyl-diphenylphosphonium; and
   benzyl-[2-(2-[¹⁸F]fluoroethoxy)phenyl]-diphenylphosphonium.
(6) The phosphonium compound according to (1), wherein the phosphonium compound is selected from:
   benzyl- [4-(2- [¹⁸F] fluoroethoxy)phenyl] -diphenylphosphonium bromide;
   benzyl-[3-(2-[¹⁸F]fluoroethoxy)phenyl]-diphenylphosphonium bromide;
   [3-(2-[¹⁸F]fluoroethoxy)phenyl]-(4-methoxyphenyl)methyl-diphenylphosphonium bromide; and
   [3-(2-[¹⁸F]fluoroethoxy)phenyl]-(3-phenylpropyl)-diphenylphosphonium bromide.
(7) A radiopharmaceutical for use in imaging, comprising the phosphonium compound according to any of (1) to (6).
(8) A radiopharmaceutical for use in PET imaging, comprising the phosphonium compound according to any of (2) to (6).
(9) The PET radiopharmaceutical according to (8), wherein the PET radiopharmaceutical is intended for use in mitochondrion imaging.
(10) The PET radiopharmaceutical according to (8) or (9), wherein the PET radiopharmaceutical is intended for use in imaging of myocardium, tumor, or brown adipose tissue.
(11) A disposable product comprising the radiopharmaceutical according to any of (7) to (10).
(12) A method for producing a quaternary phosphonium compound labeled with a radionuclide according to (1), the method comprising the step of:
   reacting an electrophile of formula (I): X¹-CH₂-A¹ with triphenylphosphine having one or more radionuclide-labeled substituents on the benzene ring to give a quaternary phosphonium salt;
   wherein X¹ is a leaving group;
   A¹ is a hydrogen atom, C₁₋₁₀alkyl optionally substituted with one or more substituents selected from B¹, C₂₋₁₀alkenyl optionally substituted with one or more substituents selected from B¹, or an aryl optionally substituted with one or more substituents selected from B²_{;}
   each B¹ is independently a halogen atom, C₁₋₆alkoxy, phenyl, or naphthyl, wherein the phenyl and naphthyl are optionally substituted with one or more substituents selected from C₁₋₆alkyl, C₁₋₆alkoxy, and halogen atoms;
   each B² is independently a halogen atom, C₁₋₆alkyl, C₁₋₆alkoxy, C₁₋₆alkylthio, cyano, amino, C₁₋₆alkylamino, di(C₁₋₆alkyl)amino, nitro, hydroxy, or (C₁₋₃alkoxy)carbonyl, wherein the alkyl, alkoxy and alkylthio are optionally substituted with one or more substituents selected from halogen atoms;
   wherein the one or more radionuclide-labeled substituents are selected from radionuclide-labeled C₁₋₆alkyl, radionuclide-labeled C₂₋₆alkoxy, radionuclide-labeled C₂₋₆alkoxyC₁₋₆alkyl, radionuclide-labeled C₂₋₆alkoxyC₂₋₆alkoxy, radionuclide-labeled C₂₋₆alkoxyC₂₋₆alkoxyC₁₋₆alkyl, and radionuclide-labeled C₂₋₆alkoxyC₂₋₆alkoxyC₂₋₆alkoxy;
   wherein the triphenylphosphine optionally further has one or more substituents selected from B² on the benzene ring.
(13) The method according to (12), wherein the radionuclide is a positron emitting nuclide.
(14) The method according to (13), wherein the positron emitting nuclide is ¹⁸F.
(15) The method according to any of (12) to (14), wherein X¹ is a halogen atom, optionally substituted C₁₋₆alkylsulfonyloxy, or optionally substituted phenylsulfonyloxy.
(16) The method according to any of (12) to (15), wherein the method is performed in an automatic synthesizer.
   Use ofa phosphonium compound as a reference standard for a labeled compound according to any one of (1) to (6), wherein the phosphonium compound corresponds to the labeled compound defined in any one of (1) to (6) except that the radionuclide of the labeled compound replaced with a non-radioactive identical element.
(20) A phosphonium compound obtained by replacing ¹⁸F in the ¹⁸F-labeled phosphonium compound according to any of (3) to (6) with ¹⁹F.
(21) Use of a phosphine compound of formula (III): for producing the phosphonium compound according to any on of claims 1 to 5 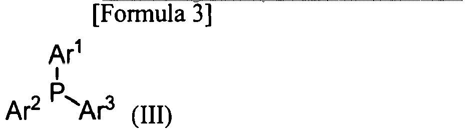
   wherein Ar¹, Ar², and Ar³ are each independently an aryl optionally substituted with one or more substituents selected from B²;
   wherein at least one of Ar¹, Ar², and Ar³ is substituted with one or more substituents selected from: C₁₋₆alkyl optionally substituted with one or more substituents selected from L, C₂₋₆alkoxy optionally substituted with one or more substituents selected from L, C₂₋₆alkoxyC₁₋₆alkyl optionally substituted with one or more substituents selected from L, C₂₋₆alkoxyC₂₋₆alkoxy optionally substituted with one or more substituents selected from L, C₂₋₆alkoxyC₂₋₆alkoxyC₁₋₆alkyl optionally substituted with one or more substituents selected from L, and C₂₋₆alkoxyC₂₋₆alkoxyC₂₋₆alkoxy optionally substituted with one or more substituents selected from L;
   B² is a halogen atom, C₁₋₆alkyl, C₁₋₆alkoxy, C₁₋₆alkylthio, cyano, amino, C₁₋₆alkylamino, di(C₁₋₆alkyl)amino, nitro, hydroxy, or (C₁₋₃alkoxy)carbonyl, wherein the alkyl, alkoxy, and alkylthio are optionally substituted with one or more substituents selected from halogen atoms;
   L is bromine, iodine, p-toluenesulfonyloxy, methanesulfonyloxy, chloromethanesulfonyloxy, or trifluoromethanesulfonyloxy.
(23) The use as set forth in (21), wherein the phosphine compounds is comprised in a kit.

### ADVANTAGEOUS EFFECTS OF INVENTION

According to the present invention, there is provided a production method that enables efficient and short-time production of quaternary phosphonium compounds labeled with a radionuclide such as ¹⁸F. Also provided according to this invention are quaternary phosphonium compounds labeled with a radionuclide such as ¹⁸F, in particular quaternary phosphonium compounds that are suitable as PET imaging agents useful for imaging of the myocardium and the like.

### BRIEF DESCRIPTION OF DRAWINGS

FIG. 1 shows the results of preparative HPLC of [¹⁸F]TAP-001 synthesized in Example 1.
FIG. 2 shows the mouse PET/CT images taken with the labeled compounds of the present invention.
FIG. 3 shows the graphs of the results of the biodistribution study conducted with the labeled compounds of the present invention.
FIG. 4 shows the graph of the results of the biodistribution study conducted with the labeled compound of the present invention.
FIG. 5 shows the PET images taken at time points from 0 to 60 minutes after administration for the purpose of conducting myocardial imaging analysis in rats using the labeled compound of the present invention.

### DESCRIPTION OF EMBODIMENTS

Hereunder, the present invention will be more specifically described.

According to one aspect of the present invention, there is provided a method for producing a quaternary phosphonium compound labeled with a positron emitting nuclide, the method comprising the step of reacting an electrophile of formula (I) given above with triphenylphosphine substituted with a positron emitting nuclide-labeled substituent.

As referred to herein, the term "aryl" refers to an aromatic hydrocarbon ring group having 6 to 14 carbon atoms. Examples of aryl include, but are not limited to, phenyl, 1-naphthyl, and 2-naphthyl.

As referred to herein, the term "C₁₋₁₀alkyl" refers to a straight-chain, branched-chain, cyclic or partially cyclic alkyl group having 1 to 10 carbon atoms. Examples of C₁₋₁₀alkyl include, but are not limited to, methyl, ethyl, n-propyl, i-propyl, n-butyl, s-butyl, i-butyl, t-butyl, n-pentyl, 3-methylbutyl, 2-methylbutyl, 1-methylbutyl, 1-ethylpropyl, n-hexyl, 4-methylpentyl, 3-methylpentyl, 2-methylpentyl, 1-methylpentyl, 3-ethylbutyl, 2-ethylbutyl, n-heptyl, n-octyl, n-nonyl, n-decyl, cyclopropyl, cyclobutyl, cyclopentyl, cyclohexyl, and cyclopropylmethyl, and other examples include, but are not limited to, C₁₋₆alkyl, C₁₋₄alkyl and C₁₋₃alkyl.

As referred to herein, the term "C₁₋₆alkyl" refers to a straight-chain, branched-chain, cyclic or partially cyclic alkyl group having 1 to 6 carbon atoms. Examples of C₁₋₆alkyl include, but are not limited to, methyl, ethyl, n-propyl, i-propyl, n-butyl, s-butyl, i-butyl, t-butyl, n-pentyl, 3-methylbutyl, 2-methylbutyl, 1-methylbutyl, 1-ethylpropyl, n-hexyl, 4-methylpentyl, 3-methylpentyl, 2-methylpentyl, 1-methylpentyl, 3-ethylbutyl, and 2-ethylbutyl, cyclopropyl, cyclobutyl, cyclopentyl, cyclohexyl, and cyclopropylmethyl, and other examples include, but are not limited to, C₁₋₄alkyl and C₁₋₃alkyl.

As referred to herein, the term "C₂₋₀alkenyl" refers to a straight-chain, branched-chain, cyclic or partially cyclic alkenyl group having 2 to 10 carbon atoms, and this alkenyl group may contain one double bond or may contain two or more double bonds. Examples of this alkenyl group include, but are not limited to, vinyl, 1-propenyl, 2-propenyl(allyl), 1-butenyl, 2-butenyl, and 3-butenyl, and other examples include, but are not limited to, C₂₋₆alkenyl, C₂₋₄alkenyl, and C₂₋₃alkenyl.

As referred to herein, the term "C₁₋₆alkoxy" refers to the alkyloxy group [-O-(C₁₋₆alkyl)] having, as an alkyl moiety, the above-defined alkyl group with 1 to 6 carbon atoms. Examples of C₁₋₆alkoxy include, but are not limited to, methoxy, ethoxy, n-propoxy, i-propoxy, n-butoxy, s-butoxy, i-butoxy, t-butoxy, n-pentoxy, 3-methylbutoxy, 2-methylbutoxy, 1-methylbutoxy, 1-ethylpropoxy, n-hexyloxy, 4-methylpentoxy, 3-methylpentoxy, 2-methylpentoxy, 1-methylpentoxy, 3-ethylbutoxy, cyclopentyloxy, cyclohexyloxy, and cyclopropylmethyloxy, and other examples include, but are not limited to, C₁₋₄alkoxy and C₁₋₃alkoxy. As referred to herein, the term "C₁₋₄alkoxy" includes, but is not limited to, C₁₋₃alkoxy.

As referred to herein, the term "C₂₋₆alkoxy" refers to the alkyloxy group [-O-(C₂₋₆alkyl)] having, as an alkyl moiety, an alkyl group with 2 to 6 carbon atoms. Examples of C₂₋₆alkoxy include, but are not limited to, ethoxy, n-propoxy, i-propoxy, n-butoxy, s-butoxy, i-butoxy, t-butoxy, n-pentoxy, 3-methylbutoxy, 2-methylbutoxy, 1-methylbutoxy, 1-ethylpropoxy, n-hexyloxy, 4-methylpentoxy, 3-methylpentoxy, 2-methylpentoxy, 1-methylpentoxy, 3-ethylbutoxy, cyclopentyloxy, cyclohexyloxy, and cyclopropylmethyloxy, and other examples include, but are not limited to, C₁₋₄alkoxy and C₁₋₃alkoxy.

As referred to herein, the term "C₁₋₆alkylthio" refers to the alkylthio group [-S-(C₁₋₆alkyl)] having, as an alkyl moiety, the above-defined alkyl group with 1 to 6 carbon atoms. Examples of C₁₋₆alkylthio include, but are not limited to, methylthio, ethylthio, n-propylthio, i-propylthio, n-butylthio, s-butylthio, i-butylthio, t-butylthio, n-pentylthio, 3-methylbutylthio, 2-methylbutylthio, 1-methylbutylthio, 1-ethylpropylthio, n-hexylthio, 4-methylpentylthio, 3-methylpentylthio, 2-methylpentylthio, 1-methylpentylthio, 3-ethylbutylthio, cyclopentylthio, cyclohexylthio, and cyclopropylmethylthio, and other examples include, but are not limited to, C₁₋₄alkylthio and C₁₋₃alkylthio. As referred to herein, the term "C₁₋₄alkylthio" includes, but is not limited to, C₁₋₃alkylthio.

As referred to herein, the term "amino" refers to -NH₂, and may form an acid addition salt in the molecule.

As referred to herein, the term "C₁₋₆alkylamino" refers to the alkylamino group [-NH-(C₁₋₆alkyl)] having, as an alkyl moiety, the above-defined alkyl group with 1 to 6 carbon atoms. Examples of C₁₋₆alkylamino include, but are not limited to, methylamino, ethylamino, n-propylamino, i-propylamino, n-butylamino, s-butylamino, i-butylamino, t-butylamino, n-pentylamino, 3-methylbutylamino, 2-methylbutylamino, 1-methylbutylamino, 1-ethylpropylamino, n-hexylamino, 4-methylpentylamino, 3-methylpentylamino, 2-methylpentylamino, 1-methylpentylamino, 3-ethylbutylamino, cyclopentylamino, cyclohexylamino, and cyclopropylmethylamino, and other examples include, but are not limited to, C₁₋₄alkylamino and C₁₋₃alkylamino. As referred to herein, the term "C₁₋₄alkylamino" includes, but is not limited to, C₁₋₃alkylamino. The C₁₋₆alkylamino group may form an acid addition salt in the molecule.

As referred to herein, the term "di(C₁₋₆alkyl)amino" refers to the alkylamino group [-N(C₁₋₆alkyl)₂] having, as an alkyl moiety, the above-defined alkyl group with 1 to 6 carbon atoms. The two alkyl groups can be the same or different. Examples of di(C₁₋₆alkyl)amino include, but are not limited to, dimethylamino, diethylamino, ethyl(methyl)amino, methyl(n-propyl)amino, ethyl(n-propyl)amino, methyl(i-propyl)amino, ethyl(i-propyl)amino, di(n-propyl)amino, and di(i-propyl)amino, and other examples include, but are not limited to, di(C₁₋₄alkyl)amino and di(C₁₋₃alkyl)amino. As referred to herein, the term "di(C₁₋₄alkyl)amino" includes, but is not limited to, di(C₁₋₃alkyl)amino. The di(C₁₋₆alkyl)amino group may form an acid addition salt in the molecule.

As referred to herein, the term "C₁₋₆alkoxycarbonyl" refers to an alkoxycarbonyl group having, as an alkoxy moiety, the above-defined C₁₋₆alkoxy group. Examples of C₁₋₆alkoxycarbonyl include, but are not limited to, methoxycarbonyl, ethoxycarbonyl, and tert-butoxycarbonyl, as well as C₁₋₃alkoxycarbonyl.

As referred to herein, the term "C₁₋₃alkoxycarbonyl" refers to an alkoxycarbonyl group having, as an alkoxy moiety, the above-defined C₁₋₃alkoxy group. Examples of C₁₋₃alkoxycarbonyl include, but are not limited to, methoxycarbonyl, ethoxycarbonyl, n-propoxycarbonyl, and isopropoxycarbonyl.

As referred to herein, the term "C₂₋₆alkoxyC₁₋₆alkyl" refers to a group of the formula -(C₁₋₆alkylene)-O-(C₂₋₆alkyl), and examples include, but are not limited to, ethoxymethyl, 2-ethoxyethyl, 2-ethoxypropyl, and 3-ethoxypropyl. Examples of ¹⁸F-labeled C₂₋₆alkoxyC₁₋₆alkyl include, but are not limited to, -CH₂OCH₂CH₂-¹⁸F, and -CH₂CH₂OCH₂CH₂-¹⁸F.

As referred to herein, the term "C₂₋₆alkoxyC₂₋₆alkoxy" refers to a group of the formula -O-(C₂₋₆alkylene)-O-(C₂₋₆alkyl), and examples include, but are not limited to, 2-ethoxyethoxy, 2-ethoxypropoxy, and 3-ethoxypropoxy. Examples of ¹⁸F-labeled C₂₋₆alkoxyC₂₋₆alkoxy include, but are not limited to, -OCH₂CH₂COH₂CH₂-¹⁸F.

As referred to herein, the term "C₂₋₆alkoxyC₂₋₆alkoxyC₁₋₆alkyl" refers to a group of the formula -(C₁₋₆alkylene)-O-(C₂₋₆alkylene)-O-(C₂₋₆alkyl), and examples include, but are not limited to, (2-ethoxyethoxy)methyl, 2-(2-ethoxyethoxy)ethyl, 2-(2-ethoxyethoxy)propyl, and 3-(2-ethoxyethoxy)propyl. Examples of ¹⁸F-labeled C₂₋₆alkoxyC₂₋₆alkoxyC₁₋₆alkyl include, but are not limited to, -CH₂OCH₂CH₂OCH₂CH₂-¹⁸F, and -CH₂CH₂OCH₂CH₂OCH₂CH₂-¹⁸F.

As referred to herein, the term "C₂₋₆alkoxyC₂₋₆alkoxyC₂₋₆alkoxy" refers to a group of the formula -O-(C₂₋₆alkylene)-O-(C₂₋₆alkylene)-O-(C₂₋₆alkyl), and examples include, but are not limited to, 2-(2-ethoxyethoxy)ethoxy, 2-(2-ethoxyethoxy)propoxy, and 3-(2-ethoxyethoxy)propoxy. Examples of ¹⁸F-labeled C₂₋₆alkoxyC₂₋₆alkoxyC2-6alkoxy include, but are not limited to, -OCH₂CH₂OCH₂CH₂OCH₂CH₂-¹⁸F.

As referred to herein, the term "optionally substituted C₁₋₆alkylsulfonyloxy" can be exemplified by methanesulfonyloxy, and trifluoromethanesulfonyloxy.

As referred to herein, the term "optionally substituted phenylsulfonyloxy" can be exemplified by benzenesulfonyloxy, and toluenesulfonyloxy.

Examples of halogen atoms include, but are not limited to, fluorine atom, chlorine atom, bromine atom, and iodine atom.

When the compound of the present invention can form a solvate such as hydrate, this invention can be practiced as a solvate. Further, the compound of the present invention can be practiced, as appropriate, in the form of mixture, solution, polymorph, or the like.

Examples of X¹ as defined herein include, but are not limited to, chlorine atom, bromine atom, iodine atom, methanesulfonyloxy, trifluoromethanesulfonyloxy, benzenesulfonyloxy, and toluenesulfonyloxy.

In one mode of the phosphonium compound of formula (II), Ar¹, Ar² and Ar³ are each independently a phenyl optionally substituted with one or more substituents selected from B².

In one mode of the phosphonium compound of formula (II), Ar¹ and Ar² are each a phenyl, and Ar³ is a phenyl optionally substituted with one or more substituents selected from B².

In one mode of the phosphonium compound of formula (II), A¹ is a hydrogen atom, C₁₋₁₀alkyl optionally substituted with one or more substituents selected from B¹, C₂₋₁₀alkenyl optionally substituted with one or more substituents selected from B¹, or a phenyl optionally substituted with one or more substituents selected from B².

The anion (X⁻) contained in the phosphonium compound is for example an anion that forms a pharmaceutically acceptable salt, and specific examples include, but are not limited to, Cl⁻, Br⁻, I⁻, PhSO₂O⁻, CH₃C₆H₄SO₂O⁻, and CH₃SO₂O⁻. In one mode, X⁻ may be an anion that is generated by the elimination of a leaving group X¹ during reaction.

Examples of the leaving group as defined herein include, but are not limited to, halogen atoms, optionally substituted C₁₋₆alkylsulfonyloxy, or optionally substituted phenylsulfonyloxy, and preferred examples include, but are not limited to, bromine atom, iodine atom, p-toluenesulfonyloxy, methanesulfonyloxy, chloromethanesulfonyloxy, and trifluoromethanesulfony loxy.

Examples of the leaving group defined herein as L or X¹ include, but are not limited to, halogen atoms, optionally substituted C₁₋₆alkylsulfonyloxy, or optionally substituted phenylsulfonyloxy, and preferred examples include, but are not limited to, bromine atom, iodine atom, p-toluenesulfonyloxy, methanesulfonyloxy, chloromethanesulfonyloxy, and trifluoromethanesulfony loxy.

In formula (II), Ar¹, Ar², Ar³ and A¹, and the substituents contained therein, when containing an amino group, an alkylamino group or a dialkylamino group, may form acid addition salts.

As used herein, the phrase "substituted with one or more substituents" means substitution with, for example, 1 to 3 substituents.

The phosphonium compound of the present invention can be contained in a variety of solutions such as aqueous solution, a variety of solvents such as hydrate, polymorphs, and the like.

The compound of the present invention can be labeled with a radionuclide by a known method. Examples of the radionuclide include, but are not limited to: ³H, ¹⁴C, ³⁵S, and ¹³¹I; γ-ray emitting nuclides such as ^{99m}Tc, ¹¹¹In, ⁶⁷Ga, ²⁰¹Tl, ¹²³I and ¹³³Xe; and positron emitting nuclides such as ¹¹C, ¹³N, ¹⁵O, ¹⁸F, ⁶²Cu, ⁶⁴Cu, ⁶⁸Ga and ⁷⁶Br. Compounds labeled with a γ-ray emitting nuclide can be used as imaging agents for computed tomography (Single photon emission computed tomography: SPECT) -- for example, compounds labeled with ^{99m}Tc and ¹²³I are commonly used for SPECT. Compounds labeled with a positron emitting nuclide can be used as imaging agents for positron emission tomography (PET). Among different positron emitting nuclides, ¹¹C, ¹³N, ¹⁵O and ¹⁸F are preferred, with ¹⁸F and ¹¹C being more preferred, and ¹⁸F being particularly preferred, from various viewpoints including appropriate half-life and ease of labeling.

The phosphonium moiety of the phosphonium compound of formula (II) can be exemplified by the following.

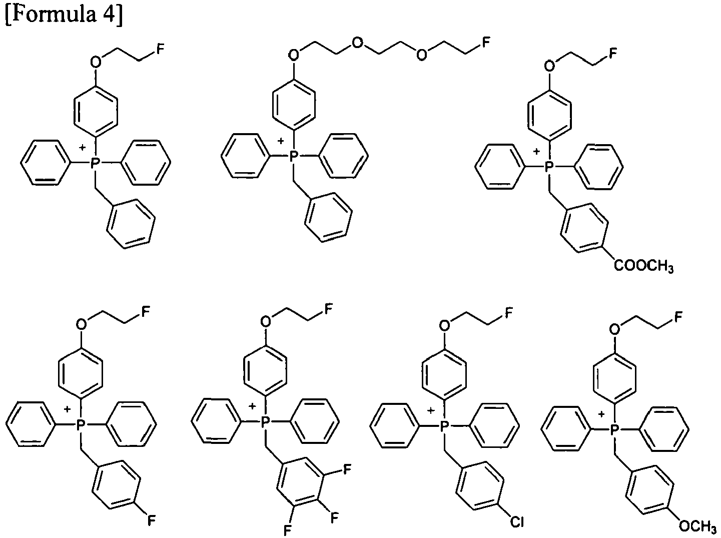

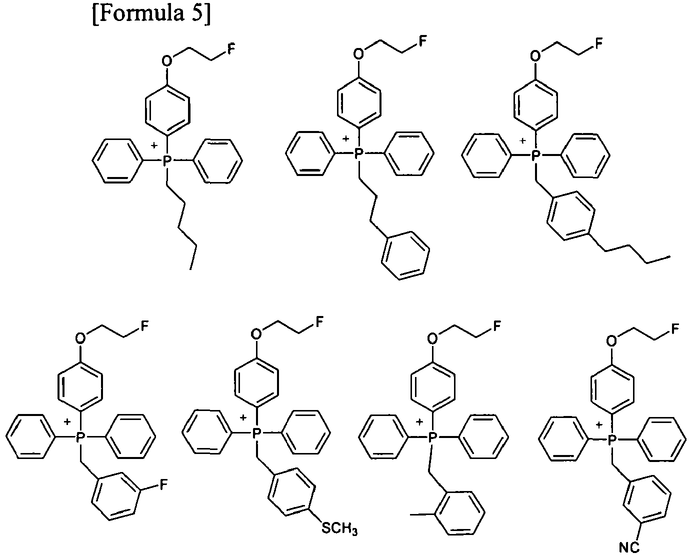

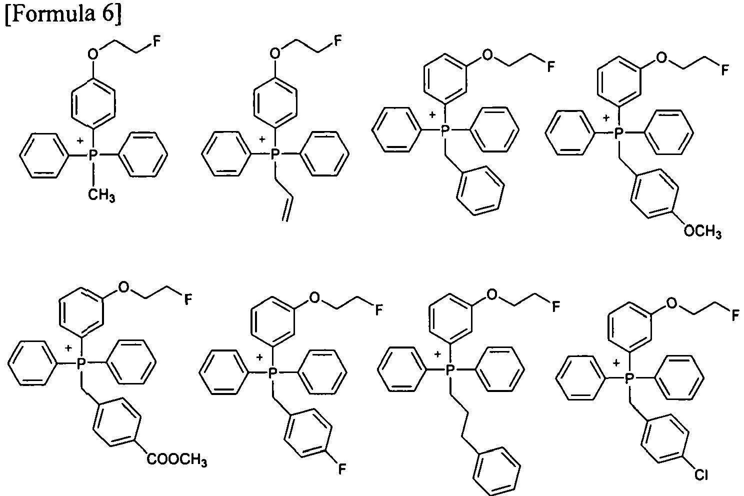

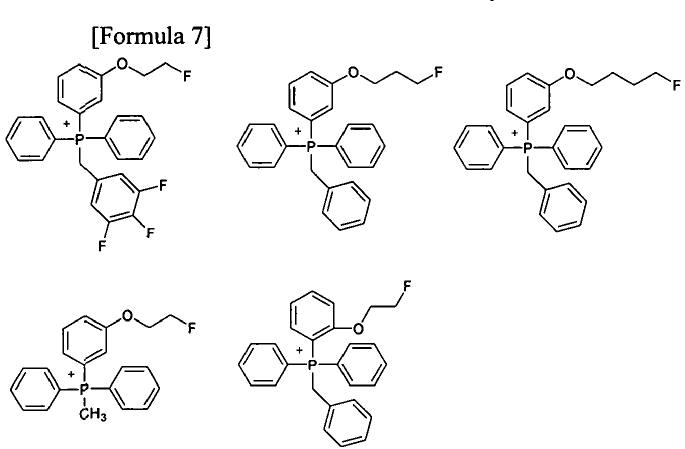

The reaction of an electrophile of formula (I) with triphenylphosphine having one or more positron emitting nuclide-labeled substituents on the benzene ring can be performed by, for example, following the step represented by the scheme given below.

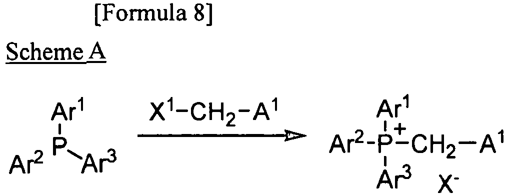

[As used in this scheme, Ar¹, Ar², Ar³, X¹, A¹, X⁻ are as defined hereinabove.]

The aforementioned reaction can be effected by performing heating (at a temperature of, for example, 50-150°C, specifically 90-120°C, more specifically 105-115°C) in the presence of an appropriate solvent (e.g., acetonitrile, DMSO, DMF, toluene, xylene). The reaction period can be set to be in the range of, for example, 5-60 minutes. This reaction can be performed, for example, under pressure by heating in a sealed container.

The production method of the present invention, together with preparation of a labeled precursor, can be performed according to the scheme given below.

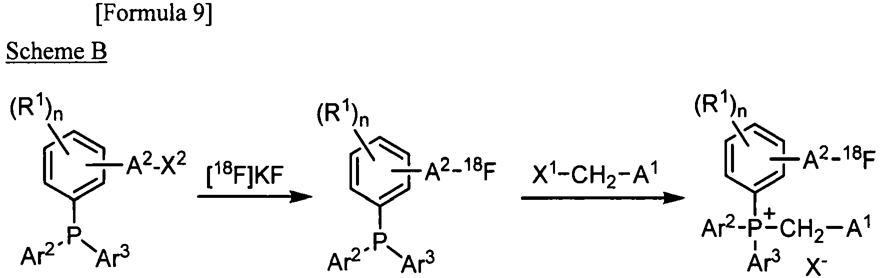

[As used in this scheme, Ar², Ar³, X¹, A¹ and X⁻ are as defined hereinabove; n is an integer selected from 1 to 4; R¹ is a group selected from B² defined above; A² is selected from -(C₁₋₆alkylene)-, -(C₁₋₆alkylene)-O-(C₂₋₆alkylene)-, -(C₁₋₆alkylene)-O-(C₂₋₆alkylene)-O-(C₂₋₆alkylene)-, -O-(C₂₋₆alkylene)-, O-O-(C₂₋₆alkylene)-O-(C₂₋₆alkylene)-, and -O-(C₂₋₆alkylene)-O-(C₂₋₆alkylene)-O-(C₂₋₆alkylene)-; X² is a leaving group.]

As [¹⁸F]KF used at the first step of fluorination, there can be used an aqueous solution of [¹⁸F]KF/K₂CO₃ which is obtained by, for example, irradiating [¹⁸O]H₂O with a proton beam using a cyclotron to thereby generate ¹⁸F⁻ and reacting ¹⁸F⁻ with K₂CO₃. The [¹⁸F] fluorination reaction can be effected by performing heating (at a temperature of, for example, 80-150°C, specifically 100-120°C, more specifically 110°C) in the presence of an appropriate solvent (e.g., acetonitrile, DMSO, DMF, DMA). The reaction period can be set to be in the range of, for example, 5-60 minutes, specifically 5-20 minutes, and more specifically set to 10 minutes. This reaction can be performed, for example, under pressure by heating in a sealed container. At the fluorination step, an additive like [2,2,2]cryptand (1,10-diaza-4,7,13,16,24,24-hexaoxabicyclo[8.8.8]hexacosane, Kryptofix® [2,2,2]) can be used to improve reaction efficiency. The aforementioned fluorination step can be performed by, for example, the following procedure: an aqueous solution of [¹⁸F]KF/K₂CO₃ and [2,2,2]cryptand are azeotropically dried to give solids, a solution of phosphine serving as a substrate is added to the solids, and then the contents are heated under sealing.

The leaving group (X²) used at the aforementioned step is a halogen atom, optionally substituted C₁₋₆alkylsulfonyloxy, or optionally substituted phenylsulfonyloxy, and preferred examples include, but are not limited to, bromine atom, iodine atom, p-toluenesulfonyloxy, methanesulfonyloxy, chloromethanesulfonyloxy, and trifluoromethanesulfonyloxy.

The second step can be performed by the same procedure as scheme A. The two steps of scheme B can be performed in a one-pot reaction: for example, after completion of the fluorination reaction, an electrophilic reagent is added to the reaction system without posttreatment and the contents are heated under sealing to obtain a product of interest.

Purification of a product of interest can be conducted by a common method: for example, purification can be done using preparative HPLC based on radiation intensity. Alternatively, purification can also be done easily using a small disposable column cartridge (Sep-Pak®) or the like.

The imaging agent of the present invention can be prepared by dissolving or suspending the positron emitting nuclide-labeled quaternary phosphonium compound into physiological saline or the like. The imaging agent may contain any additives such as pH adjustor, resolvent, dispersant, solubilizer, and radical scavenger (radiolysis inhibitor) depending on the need.

The dose of the imaging agent of this invention can be selected, as appropriate, depending on various factors including the organ to be imaged and the body shape of a subject (patient). The imaging agent of this invention can comprise a therapeutically and/or prophylactically effective amount of the compound of formula (I) given above. In this invention, the compound of formula (I) can be generally used at a dose of 0.001 mg/body kg.

The imaging agent of the present invention can be used for detection of tumor cells by PET and monitoring of the circulatory system, especially the myocardium. Therefore, the inventive imaging agent can be used for the purposes of: examination of tumors such as brain tumor, head and neck cancer, lung cancer and liver cancer; and diagnosis of ischemic heart diseases such as *angina pectoris,* myocardial infarction, arteriosclerosis of coronary artery, etc., and myocardial ischemia.

### EXAMPLES

Hereunder, the present invention will be described in more detail by way of working examples, but this invention is not limited to these examples. Additionally, in the Hi-flash silica column chromatography conducted for purification of the compounds synthesized in the working examples given below, the flash chromatography system YFLC-AI-700 and the Hi-flash columns (silica gel) produced by Yamazen Corporation were used. Mass spectroscopy was done using JMS-700 produced by JEOL Ltd. or LCMS-2020 produced by Shimadzu Corporation. High performance liquid chromatography (HPLC) was done using the LC-2000Plus system produced by JASCO Corporation.

### Synthesis Example 1: 4-Diphenylphosphanyl phenol

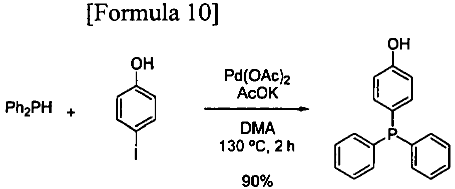

A reaction vessel was charged with 4-iodophenol (5.90 g, 26.9 mmol), potassium acetate (3.17 g, 32.3 mmol), palladium acetate (II) (36.0 mg, 0.6 mol%), and N,N-dimethylacetamide (27 mL), and while the contents were stirred, diphenylphosphine (4.63 mL, 26.9 mmol) was dropwise added slowly. Then, the reaction vessel was heated to 130°C, and reaction was effected for 2 hours. After the reaction, water was added to the reaction system, and the product was extracted three times with CH₂Cl₂ into the organic layer. The resulting organic layer was washed with saturated saline and dried with MgSO₄. After concentration under reduced pressure, the concentrate was purified by Hi-flash silica column chromatography (developing solvent, AcOEt:hexane = 11:89 --> 32:68) to yield a product (6.74 g (24.2 mmol), 90% yield, white solid).
¹H-NMR (600 MHz, CDCl₃): δ7.36-7.15 (m, 12H), 6.82 (d, J = 7.8 Hz, 2H), 5.01 (s, 1H);
LRMS (EI) calculated for C₁₈H₁₅OP (M⁺): 278.1, found: 278.1.

### Synthesis Example 2: Toluene-4-sulfonic acid 2-(4-diphenylphosphanyl-phenoxy)-ethyl ester

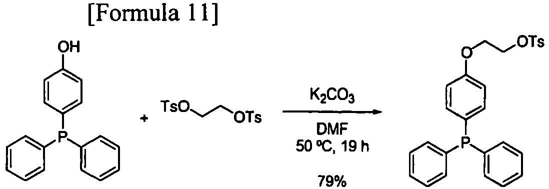

A reaction vessel was charged with 4-diphenylphosphanyl phenol (434 mg, 1.56 mmol), 1,2-bis(tosyloxy)ethane (2.89 g, 7.80 mmol), potassium carbonate (431 mg, 3.12 mmol), and N,N-dimethylformamide (DMF, 5 mL). The reaction vessel was heated to 50°C, and reaction was effected for 19 hours. After the reaction, water and ethyl acetate were added to the reaction system, unreacted 1,2-bis(tosyloxy)ethane was removed by filtration, and then the product was extracted three times with ethyl acetate into the organic layer. The resulting organic layer was washed with saturated saline and dried with MgSO₄. After concentration under reduced pressure, the concentrate was separated by Hi-flash silica column chromatography (developing solvent, ethyl acetate:hexane = 22:78 --> 43:57) to yield a product (587 mg (1.23 mmol), 79% yield, white solid).
¹H-NMR (600 MHz, CDCl₃): δ7.81 (d, J = 7.8 Hz, 2H), 7.34-7.31 (m, 8H), 7.29-7.21 (m, 6H), 6.77 (d, J = 7.8 Hz, 2H), 4.38-4.35 (m, 2H), 4.16-4.14 (m, 2H), 2.43 (s, 3H);
LRMS (EI) calculated for C₂₇H₂₅O₄PS (M⁺): 476.1, found: 476.1.

### Synthesis Example 3: 2-Diphenylphosphanyl phenol

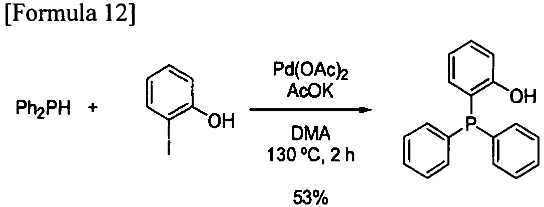

The same synthesis procedure as in Synthesis Example 1 was performed except that 4-iodophenol was replaced by 2-iodophenol. Separation was done by Hi-flash silica column chromatography (developing solvent, ethyl acetate:hexane = 18:82 --> 39:61) to yield a product (53% yield, white solid).
¹H-NMR (600 MHz, CDCl₃): δ7.38-7.28 (m, 11H), 7.00-6.87 (m, 3H), 6.16 (d, J = 6.6, Hz, 1H);
LRMS (EI) calculated for C₁₈H₁₅OP (M⁺): 278.1, found: 278.1.

### Synthesis Example 4: Toluene-4-sulfonic acid 2-(2-diphenylphosphanyl-phenoxy)-ethyl ester

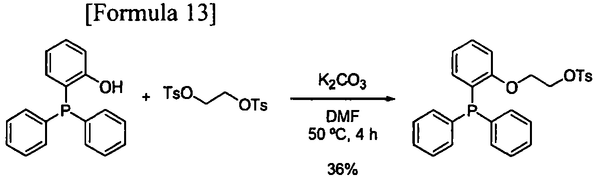

The same synthesis procedure as in Synthesis Example 2 was performed using compound 5 as a starting material. Separation was done by Hi-flash silica column chromatography (developing solvent, ethyl acetate:hexane = 22:78 --> 43:57) to yield a product (36% yield, white solid).
LRMS (EI) calculated for C₂₇H₂₅O₄PS (M⁺): 476.1, found: 476.0.

### Synthesis Example 5: 3-Diphenylphosphanyl phenol

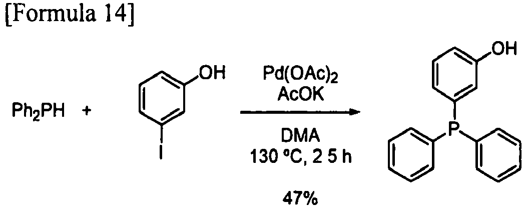

The same synthesis procedure as in Synthesis Example 1 was performed except that 4-iodophenol was replaced by 3-iodophenol. Separation was done by Hi-flash silica column chromatography (developing solvent, ethyl acetate:hexane = 12:88 --> 33:67) to yield a product (47% yield, white solid).
¹H-NMR (600 MHz, CDCl₃): δ7.37-7.28 (m, 10H), 7.22 (td, J = 7.8, 1.8 Hz, 1H), 6.90 (t, J = 7.8 Hz, 1H), 6.81 (dd, J = 7.8, 2.4 Hz, 1H), 6.70 (dt, J = 7.2, 1.2 Hz, 1H), 4.82 (s, 1H);
LRMS (EI) calculated for C₁₈H₁₅OP (M⁺): 278.1, found: 278.1.

### Synthesis Example 6: Toluene-4-sulfonic acid 2-(3-diphenylphosphanyl-phenoxy)-ethyl ester

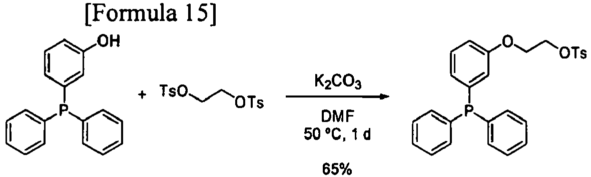

The same synthesis procedure as in Synthesis Example 2 was performed using compound 8 as a starting material. Separation was done by Hi-flash silica column chromatography (developing solvent, ethyl acetate:hexane = 22:78 --> 43:57) to yield a product (65% yield, white solid).
¹H-NMR (600 MHz, CDCl₃): δ7.78 (d, J = 7.8 Hz, 2H), 7.37-7.25 (m, 13H), 6.87 (t, J = 7.8 Hz, 1H), 6.75 (d, J = 7.8 Hz, 1H), 6.72 (d, J = 7.8 Hz, 1H), 4.34-4.29 (m, 2H), 4.06-4.03 (m, 2H), 2.42 (s, 3H);
LRMS (EI) calculated for C₂₇H₂₅O₄PS (M⁺): 476.1, found: 476.1.

### Synthesis Example 7: Toluene-4-sulfonic acid 3-phenylpropyl ester

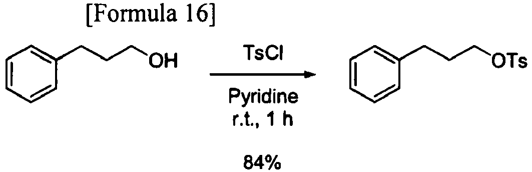

A reaction vessel was charged with 3-phenyl-1-propanol (500 µL, 3.67 mmol), p-toluenesulfonylchloride (770 mg, 4.04 mmol), and pyridine (1 mL), and reaction was effected at room temperature for 1 hour. After the reaction, water and hydrochloric acid were added to the reaction system, and the product was extracted three times with ethyl acetate into the organic layer. The resulting organic layer was washed with an aqueous solution of saturated sodium bicarbonate and dried wtih MgSO₄. After concentration under reduced pressure, the concentrate was purified by Hi-flash silica column chromatography (developing solvent, ethyl acetate:hexane = 22:78 --> 43:57) to yield a product (898 mg (3.09 mmol), 84% yield, colorless liquid).
¹H-NMR (600 MHz, CDCl₃): δ7.79 (d, J = 7.8 Hz, 2H), 7.34 (d, J = 7.8 Hz, 2H), 7.24 (t, J = 7.8 Hz, 2H), 7.17 (t, J = 7.8 Hz, 1H), 7.06 (d, J = 7.8 Hz, 2H), 4.03 (t, J = 6.0 Hz, 2H), 2.65 (t, J = 7.2 Hz, 2H), 2.46 (s, 3H), 1.99-1.93 (m, 2H);
LRMS (EI) calculated for C₁₆H₁₈O₃S (M⁺): 290.1, found: 290.1.

### Synthesis Example 8: 3-Iodopropylbenzene

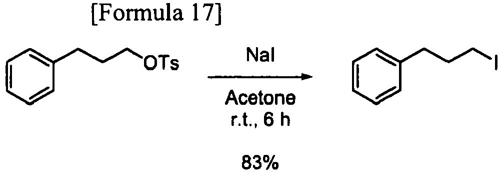

A reaction vessel was charged with 3-(4-methylphenylsulfonyloxy)propylbenzene (200 mg, 0.688 mmol), sodium iodide (310 mg, 2.07 mmol), and acetone (3.4 mL), and reaction was effected at room temperature for 6 hours. After the reaction, water was added to the reaction system, and the product was extracted three times with ethyl acetate into the organic layer. The resulting organic layer was washed with saturated saline and dried with MgSO₄. After concentration under reduced pressure, the concentrate was purified by Hi-flash silica column chromatography (developing solvent, ethyl acetate:hexane = 0:100 --> 11:89) to yield a product (140 mg (0.569 mmol), 83% yield, orange liquid).
¹H-NMR (600 MHz, CDCl₃): δ7.29 (t, J = 7.8 Hz, 2H), 7.22 (d, J = 7.8 Hz, 1H), 7.20 (d, J = 7.8 Hz, 2H), 3.18 (t, J = 7.2 Hz, 2H), 2.73 (t, J = 7.2 Hz, 2H), 2.14 (quint, J = 7.2 Hz, 2H);
LRMS (EI) calculated for C₉H₁₁I (M⁺): 246.0, found: 246.0.

As a reference specimen for use in the identification of labeled compounds, an unlabeled compound was synthesized according to the procedure described below.

### Synthesis Example 9: Toluene-4-sulfonic acid 2-fluoroethyl ester

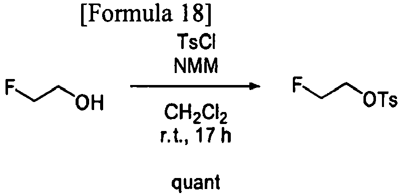

A reaction vessel was charged with 2-fluoroethanol (1.00 mL, 16.9 mmol), p-toluenesulfonylchloride (3.85 g, 20.2 mmol), N-methylmorpholine (9.30 mL, 84.5 mmol), and CH₂Cl₂ (50 mL), and reaction was effected at room temperature for 17 hours. After the reaction, water was added to the reaction system, and the product was extracted three times with ethyl acetate into the organic layer. The resulting organic layer was washed with saturated saline and dried with MgSO₄. After concentration under reduced pressure, the concentrate was purified by Hi-flash silica column chromatography (developing solvent, ethyl acetate:hexane = 19:81 --> 40:60) to yield a product (3.79 g (17.2 mmol), >100% yield, light yellow liquid).
¹H-NMR (600 MHz, CDCl₃): δ7.81 (d, J = 8.4 Hz, 2H), 7.36 (d, J = 8.4 Hz, 2H), 4.63-4.60 (m, 1H), 4.55-4.52 (m, 1H), 4.31-4.27 (m, 1H), 4.26-4.23 (m, 1H), 2.46 (s, 3H);
LRMS (EI) calculated for C9H₁₁FO₃S (M⁺): 218.0, found: 218.0.

### Synthesis Example 10: [4-(2-Fluoroethoxy)phenyl]-diphenylphosphine

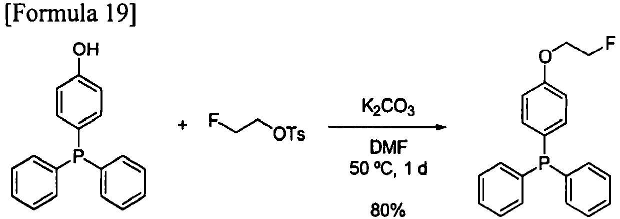

A reaction vessel was charged with 4-diphenylphosphanyl phenol (1.00 g, 3.59 mmol), toluene-4-sulfonic acid 2-fluoroethyl ester (1.02 g, 4.67 mmol), potassium carbonate (992 mg, 7.18 mmol), and DMF (5 mL). The reaction vessel was heated to 50°C, and reaction was effected for 1 day. After the reaction, water was added to the reaction system, and the product was extracted three times with ethyl acetate into the organic layer. The resulting organic layer was washed with saturated saline and dried with MgSO₄. After concentration under reduced pressure, the concentrate was purified by Hi-flash silica column chromatography (developing solvent, ethyl acetate:hexane = 4:96 --> 25:75) to yield a product (921 mg (2.84 mmol), 80% yield, white solid).
¹H-NMR (600 MHz, CDCl₃): δ7.69-7.43 (m, 1H), 7.36-7.24 (m, 11H), 7.01-6.89 (m, 2H), 4.75 (dt, J = 48.0, 4.2 Hz, 2H), 4.21 (dt, J = 28.2, 4.2 Hz, 2H);
LRMS (EI) calculated for C₂₀H₁₈FOP (M⁺): 324.1, found: 324.1.

### Synthesis Example 11: Benzyl-[4-(2-fluoroethoxy)phenyl]-diphenylphosphonium bromide (TAP-001)

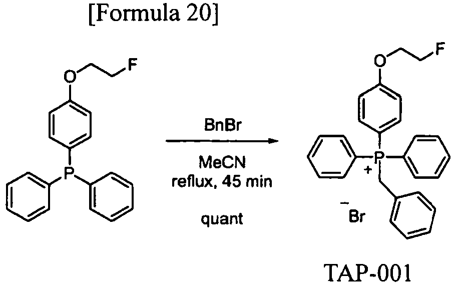

A reaction vessel was charged with [4-(2-fluoroethoxy)phenyl]-diphenylphosphine (100 mg, 0.308 mmol), benzyl bromide (366 µL, 3.08 mmol), and MeCN (2 mL). The reaction vessel was heated to 100°C, and the contents were refluxed under heating for 45 minutes. After the reaction, the reaction solution was introduced into an open silica gel column chromatography system to elute unreacted benzyl bromide with ethyl acetate. Then, with the developing solvent being replaced by methanol:CH₂Ch = 25:75, separation was done to yield a product (161 mg (0.326 mmol), 106% yield, white solid).
¹H-NMR (600 MHz, CDCl₃): δ7.78-7.70 (m, 4H), 7.69-7.59 (m, 8H), 7.25-7.07 (m, 7H), 5.26 (d, J = 14.4 Hz, 2H), 4.80 (dt, J = 48.0, 4.2 Hz, 2H), 4.35 (dt, J = 28.2, 4.2 Hz, 2H);
LRMS (FAB) calculated for C₂₇H₂₅FOP⁺ ([M-Br]⁺): 415.2, found: 415.2 ([M-Br]⁺).

Likewise, following the same synthesis procedure, the compounds shown below were also synthesized by reacting [4-(2-fluoroethoxy)phenyl]-diphenylphosphine with an electrophile.

### [Table 1-1]

**Table 1**

| Compound | Structure | Yield | Property | MS |
|---|---|---|---|---|
| TAP-002 | 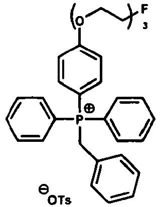 | 38% | White solid | LRMS (FAB) calculated for C₃₁ H₃₃FO₃P⁺ ([M-OTs]⁺): 503.2, |
| | | | | found: 503.2 ([M-OTs]⁺) |
| TAP-003 | 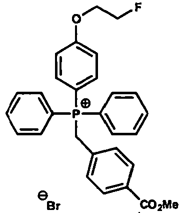 | 90% | Colorless solid | LRMS (ESI) calculated for C₂₉H₂₇FO₃P⁺ ([M-Br]⁺): 473.1 , |
| | | | | found: 473.1 ([M-Br]⁺) |
| TAP-004 | 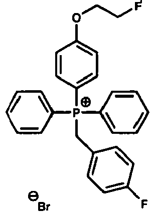 | 99% | White solid | LRMS (ESI) calculated for C₂₇H₂₄F₂OP⁺ ([M-Br]⁺): 433.1, |
| | | | | found: 433.1 ([M-Br]⁺) |
| TAP-005 | 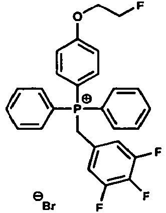 | 85% | Colorless solid | LRMS (ESI) calculated for C₂₇H₂₂FO₄P⁺ ([M-Br]⁺): 469.1, |
| | | | | found: 469.1 ([M-Br]⁺) |
| TAP-006 | 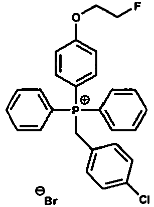 | 104% (containing MeOH) | White solid | LRMS (ESI) calculated for C₂₇H₂₄CIFOP⁺ ([M-Br]⁺): 449.1, |
| | | | | found: 449.1 ([M-Br]⁺) |
| TAP-007 | 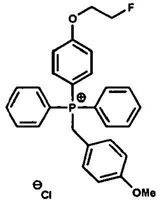 | 90% | White solid | LRMS (ESI) calculated for C₂₈H₂₇FO₂P⁺ ([M-Cl]+): 445.1, |
| | | | | found: 445.1 ([M-Cl]+) |

**[Table 1-2]**

| | | | | |
|---|---|---|---|---|
| TAP-008 | 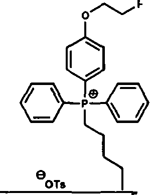 | 87% | Colorless gum | LRMS (ESI) calculated for C₂₅H₂₉FOP⁺ ([M-OTs]⁺): 395.1, |
| | | | | found: 395.1 ([M-OTs]⁺) |
| TAP-010 | 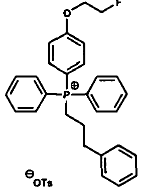 | 56% | Colorless solid | LRMS (ESI) calculated for C₂₉H₂₉FOP⁺ ([M-OTs]⁺): 443.1, |
| | | | | found: 443.1 ([M-OTs]⁺) |
| TAP-011 | 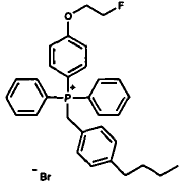 | 87% | Colorless solid | LRMS (ESI) calculated for C₃₁H₃₃FOP⁺([M-Br]⁺): 471.2, |
| | | | | found: 471.2 ([M-Br]⁺) |
| TAP-012 | 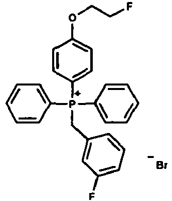 | 85% | Colorless solid | LRMS (ESI) calculated for C₃₁H₃₃FOP⁺ ([M-Br]⁺): 433.1, |
| | | | | found: 433.1 ([M-Br]⁺) |
| TAP-013 | 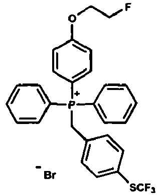 | 89% | Colorless solid | LRMS (ESI) calculated for C₃₁H₃₃FOP⁺ ([M-Br]⁺): 515.1, |
| | | | | found: 515.1 ([M-Br]⁺) |
| TAP-014 | 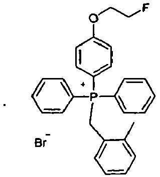 | 89% | Colorless solid | LRMS (ESI) calculated for C₃₁H₃₃FOP⁺([M-Br]⁺): 429.1, |
| | | | | found: 429.1 ([M-Br]⁺) |
| TAP-015 | 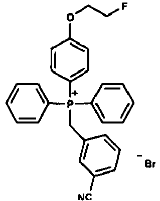 | 88% | White solid | LRMS (ESI) calculated for C₃₁H₃₃FOP⁺ ([M-Br]⁺): 440.1, |
| | | | | found: 440.1 ([M-Br]⁺) |

**[Table 1-3]**

| | | | | |
|---|---|---|---|---|
| TAP-016 | 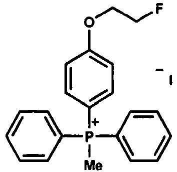 | 77% | Colorless solid | LRMS (ESI) calculated for C₃₁H₃₃FOP⁺([M-1]⁺): 339.1, |
| | | | | found: 339.0 ([M-I]⁺) |
| TAP-017 | 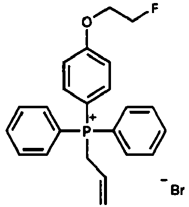 | 85% | Colorless solid | LRMS (FAB) calculated for C₂₃H₂₃FOP⁺ ([M-Br]⁺): 365.1, |
| | | | | found: 365.2 ([M-Br]⁺) |

### Synthesis Example 12: [2-(2-Fluoroethoxy)phenyl]-diphenylphosphine

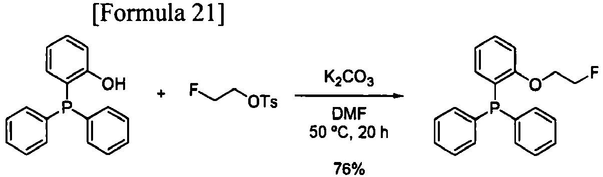

The same synthesis procedure as in Synthesis Example 10 was performed using 2-diphenylphosphanyl phenol as a starting material. Separation was done by Hi-flash silica column chromatography (developing solvent, ethyl acetate:hexane = 3:97 --> 21:79) to yield a product (76% yield, white solid).
¹H-NMR (600 MHz, CDCl₃): δ7.36-7.29 (m, 11H), 6.91-6.87 (m, 2H), 6.74-6.70 (m, 1H), 4.40 (dt, J = 47.4, 4.8 Hz, 2H), 4.11 (dt, J = 25.8, 4.8 Hz, 2H);
LRMS (EI) calculated for C₂₀H₁₈FOP (M⁺): 324.1, found: 324.1.

### Synthesis Example 13: Benzyl-[2-(2-fluoroethoxy)phenyl]-diphenylphosphonium bromide (TAP-028)

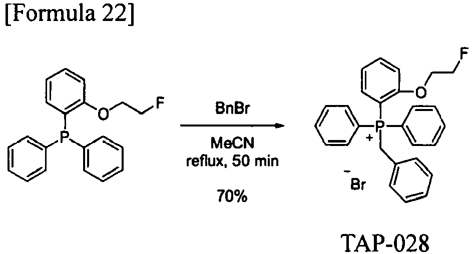

The same synthesis procedure as in Synthesis Example 11 was performed using [2-(2-fluoroethoxy)phenyl]-diphenylphosphine as a starting material. Unreacted benzyl bromide was eluted with ethyl acetate, and then, with the developing solvent being replaced by methanol:CH₂Cl₂ = 25:75, separation was done to yield a product (70% yield, white solid).
¹H-NMR (600 MHz, CDCl₃): δ7.90-7.83 (m, 1H), 7.80-7.73 (m, 2H), 7.66-7.58 (m, 4H), 7.55-7.46 (m, 5H), 7.35-7.25 (m, 2H), 7.23-7.16 (m, 3H), 7.01 (d, J = 6.6 Hz, 1H), 5.03 (d, J = 15.0 Hz, 2H), 4.51 (s, 2H), 4.48-4.46 (m, 1H), 4.44-4.42 (m, 1H);
LRMS (FAB) calculated for C₂₇H₂₅FOP⁺ ([M-Br]⁺): 415.2, found: 415.2 ([M-Br]⁺).

### Synthesis Example 14: [3-(2-Fluoroethoxy)phenyl]-diphenylphosphine

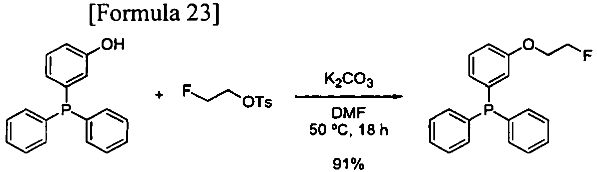

The same synthesis procedure as in Synthesis Example 10 was performed using 3-diphenylphosphanyl phenol as a starting material. Separation was done by Hi-flash silica column chromatography (developing solvent, ethyl acetate:hexane = 4:96 --> 25:75) to yield a product (91% yield, white solid).
¹H-NMR (600 MHz, CDCl₃): δ7.41-7.24 (m, 11H), 6.94-6.84 (m, 3H), 4.77-4.71 (m, 1H), 4.69-4.63 (m, 1H), 4.24-4.08 (m, 2H);
LRMS (EI) calculated for C₂₀H₁₈FOP (M⁺): 324.1, found: 324.1.

### Synthesis Example 15: Benzyl-[3-(2-fluoroethoxy)phenyl]-diphenylphosphonium bromide (TAP-018)

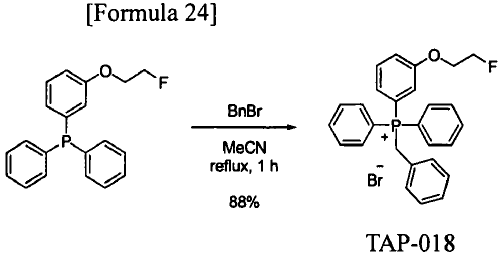

The same synthesis procedure as in Synthesis Example 11 was performed using [3-(2-fluoroethoxy)phenyl]-diphenylphosphine as a starting material. Unreacted benzyl bromide was eluted with ethyl acetate, and then, with the developing solvent being replaced by methanol:CH₂Cl₂ = 20:80, separation was done to yield a product (88% yield, white solid).
¹H-NMR (600 MHz, CDCl₃): δ7.80-7.74 (m, 3H), 7.70-7.64 (m, 4H), 7.63-7.58 (m, 4H), 7.50-7.46 (m, 1H), 7.35-7.31 (m, 1H), 7.24-7.20 (m, 1H), 7.16-7.09 (m, 5H), 5.49 (d, J = 14.4 Hz, 2H), 4.84-4.80 (m, 1H), 4.76-4.72 (m, 1H), 4.57-4.54 (m, 1H), 4.52-4.49 (m, 1H);
LRMS (FAB) calculated for C₂₇H₂₅FOP⁺ ([M-Br]⁺): 415.2, found: 415.2 ([M-Br]⁺).

### Synthesis Example 16: [3-(2-Fluoroethoxy)phenyl]-(4-methoxycarbonylphenyl)methyl-diphenylphosphonium bromide (TAP-020)

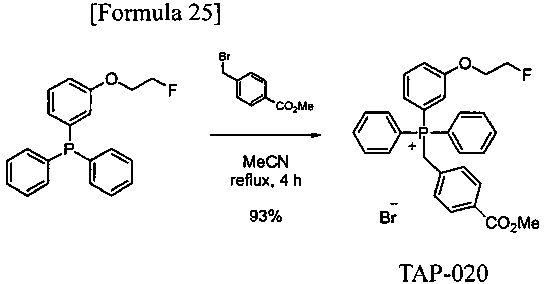

A reaction vessel was charged with [3-(2-fluoroethoxy)phenyl]-diphenylphosphine (63.8 mg, 0.197 mmol), 4-(bromomethyl)benzoic acid methyl ester (54.8 mg, 0.239 mmol), and acetonitrile (2 mL). The reaction vessel was heated to 90°C, and the contents were refluxed under heating for 4 hours. After the reaction, the reaction solution was introduced into an open silica gel column chromatography system to elute an unreacted compound with ethyl acetate. Then, with the developing solvent being replaced by methanol:CH₂Cl₂ = 20:80, separation was done to yield a product (102 mg (0.184 mmol), 93% yield, white solid).
¹H-NMR (600 MHz, CDCl₃): δ7.85 (d, J = 14.4 Hz, H), 7.79-7.74 (m, 4H), 7.70 (d, J = 14.4 Hz, 2H), 7.68 (d, J = 14.4 Hz, 2H), 7.63-7.58 (m, 4H), 7.46 (td, J = 7.8, 4.2 Hz, 1H), 7.32 (d, J = 7.8 Hz, 1H), 7.23-7.20 (m, 2H), 7.11 (dd, J = 13.2, 7.2 Hz, 1H), 5.71 (d, J = 15.0 Hz, 2H), 4.77 (dt, J = 47.4, 3.6 Hz, 2H), 4.54 (dt, J = 28.8, 3.6 Hz, 2H), 3.86 (s, 3H);
LRMS (FAB) calculated for C₂₉H₂₇FO₃P⁺ ([M-Br]): 473.2, found: 473.2 ([M-Br]).

### Synthesis Example 17: [3-(2-Fluoroethoxy)phenyl]-(4-methoxyphenyl)methyl-diphenylphosphonium bromide (TAP-019)

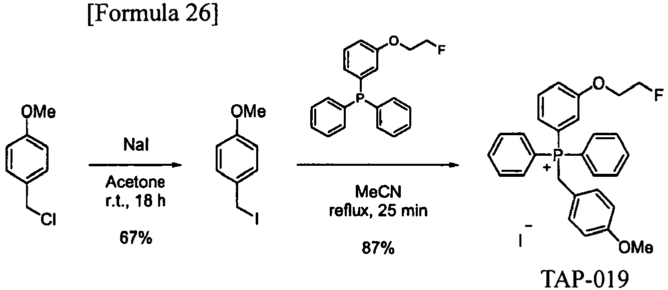

A reaction vessel was charged with 4-methoxybenzylchloride (431 µL, 3.19 mmol), sodium iodide (1.44 g, 9.58 mmol), and acetone (16 mL), and reaction was effected at room temperature for 18 hours. After the reaction, water was added to the reaction system, and the product was extracted three times with ethyl acetate into the organic layer. The resulting organic layer was washed with saturated saline and dried with MgSO₄. After concentration under reduced pressure, the concentrate was purified by Hi-flash silica column chromatography (developing solvent, ethyl acetate:hexane = 1:99 --> 20:80) to yield 4-methoxybenzyl iodide (527 mg (2.12 mmol), 67% yield, yellow liquid).

Subsequently, the reaction vessel was charged with the resulting 4-methoxybenzyl iodide (57.3 mg, 0.231 mmol), [3-(2-fluoroethoxy)phenyl]-diphenylphosphine (50.0 mg, 0.154 mmol), and acetonitrile (2 mL). The reaction vessel was heated to 100°C, and the contents were refluxed under heating for 25 minutes. After the reaction, the reaction solution was introduced into an open silica gel column chromatography system to elute an unreacted compound with ethyl acetate. Then, with the developing solvent being replaced by methanol:CH₂Cl₂ = 10:90, separation was done to yield a product (76.3 mg (0.133 mmol), 86.6% yield, light yellow solid).
¹H-NMR (600 MHz, CDCl₃): δ7.79 (t, J = 7.2 Hz, 2H), 7.70-7.61 (m, 8H), 7.52 (td, J = 8.4, 4.2 Hz, 1H), 7.46 (d, J = 13.2 Hz, 1H), 7.63-7.58 (m, 4H), 7.46 (td, J = 7.8, 4.2 Hz, 1H), 7.32 (d, J = 9.6 Hz, 1H), 7.14 (dd, J = 12.3, 7.2 Hz, 1H), 7.02 (d, J = 7.2 Hz, 1H), 6.67 (d, J = 8.4 Hz, 2H), 5.22 (d, J = 13.8 Hz, 2H), 4.77 (dt, J = 46.5, 3.6 Hz, 2H), 4.46 (dt, J = 30.0, 3.6 Hz, 2H), 3.73 (s, 3H);
LRMS (FAB) calculated for C₂₈H₂₇FIO₂P⁺ ([M-I]⁺): 445.2, found: 445.2 ([M-I]⁺).

### Synthesis Example 18: [3-(2-Fluoroethoxy)phenyl]-diphenyl-(3-phenylpropyl)phosphonium bromide (TAP-022)

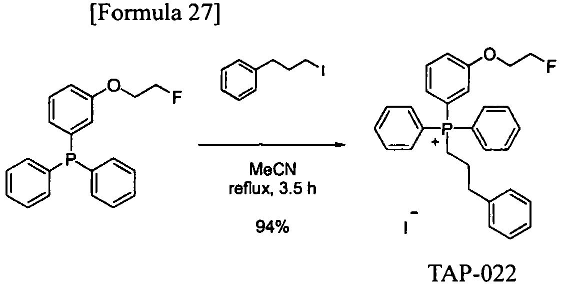

A reaction vessel was charged with [3-(2-fluoroethoxy)phenyl]-diphenylphosphine (50.0 mg, 0.154 mmol), 3-iodopropylbenzene (76.0 mg, 0.308 mmol), and acetonitrile (1 mL). The reaction vessel was heated to 100°C, and the contents were refluxed under heating for 3.5 hours. After the reaction, the reaction solution was introduced into an open silica gel column chromatography system to elute an unreacted compound with ethyl acetate. Then, with the developing solvent being replaced by methanol:CH₂Cl₂ = 20:80, separation was done to yield a product (73.5 mg (0.145 mmol), 94% yield, light yellow solid).
¹H-NMR (600 MHz, CDCl₃): δ7.81-7.77 (m, 2H), 7.74-7.69 (m, 4H), 7.68-7.64 (m, 4H), 7.54 (td, J = 7.8, 4.2 Hz, 1H), 7.50-7.46 (m, 1H), 7.34-7.32 (m, 1H), 7.29-7.25 (m, 2H), 7.22-7.16 (m, 4H), 4.79 (dt, J = 36.6, 4.2 Hz, 2H), 4.48 (dt, J = 28.8, 4.2 Hz, 2H), 3.89-3.83 (m, 2H), 3.05 (t, J = 7.2 Hz, 2H), 2.01-1.93 (m, 2H);
LRMS (FAB) calculated for C₂₉H₂₉FIOP⁺ ([M-I]⁺): 443.2, found: 443.2 ([M-I]⁺). Likewise, following the same synthesis procedure, the compounds shown below were synthesized by reacting [3-(2-fluoroethoxy)phenyl]-diphenylphosphine, [3-(3-fluoropropoxy)phenyl]-diphenylphosphine, or [3-(4-fluorobutoxy)phenyl]-diphenylphosphine with an electrophile.

### [Table 2]

**Table 2**

| Compound | Structure | Yield | Property | MS |
|---|---|---|---|---|
| TAP-021 | 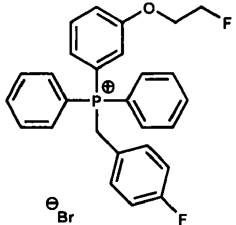 | 92% | White solid | LRMS (ESI) calculated for C₂₇H₂₄F₂OP⁺ ([M-Br]⁺): 433.1, |
| | | | | found: 433.0 ([M-Br]⁺) |
| TAP-023 | 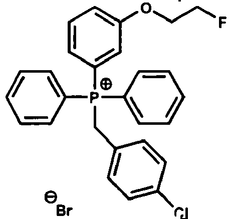 | 90% | Colorless solid | LRMS (ESI) calculated for C₂₇H₂₄ClFOP⁺ ([M-Br]⁺): 449.1, |
| | | | | found: 449.0 ([M-Br]⁺) |
| TAP-024 | 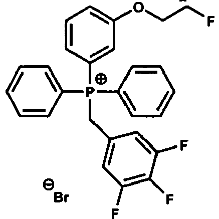 | 55% | White solid | LRMS (ESI) calculated for C₂₇H₂₂F₄OP⁺ ([M-Br]⁺): 469.1, |
| | | | | found: 469.1 ([M-Br]⁺) |
| TAP-025 | 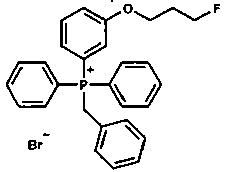 | 91% | White solid | LRMS (FAB) calculated for C₂₇H₂₂F₄OP⁺ ([M-Br]⁺): 429.1, |
| | | | | found: 429.2 ([M-Br]⁺) |
| TAP-026 | 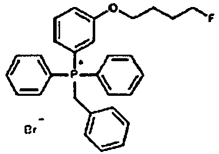 | 97% | White solid | LRMS (FAB) calculated for C₂₆H₂₉FOP⁺ ([M-Br]⁺): 443.1, |
| | | | | found: 443.2 ([M-Br]⁺) |
| TAP-027 | 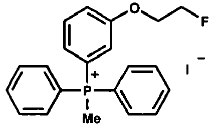 | 71% | White solid | LRMS (FAB) calculated for C₂₁H₂₁FOP⁺ ([M-I]⁺): 339.1, |
| | | | | found: 339.2 ([M-I]⁺) |

### Procedure for ¹⁸F-labeling reaction

A 12 MeV proton beam accelerated by the cyclotron Cypris HM12 (produced by Sumitomo Heavy Industries, Ltd.) was irradiated to [¹⁸O]H₂O with an isotropic purity of 97% or higher (produced by Taiyo Nippon Sanso Corporation) for 40 minutes, thereby effecting an ¹⁸O(p,n)¹⁸F nuclear reaction to synthesize ¹⁸F⁻. Then, the solution was passed through a cation exchange resin (AG1-X8) to trap ¹⁸F⁻ on the resin. The trapped ¹⁸F⁻ was eluted with a 33 mM solution of K₂CO₃ to give an aqueous solution of [¹⁸F]KF/K₂CO₃. An appropriate amount of radioactivity (55-65 mCi) of the aqueous solution was taken in a volume of about 150-250 µL, and added to a reaction vial containing 800 µL of a Kryptfix® 2.2.2. MeCN solution (20 mg/mL), and the contents were azeotropically dried under stream of helium in an oil bath at 100°C. Then, 600 µL each of the prepared different MeCN solutions of labeling precursor {3 mg (6.30 µmol)/600 µL} was added to the reaction vial, and an ¹⁸F fluorination reaction was performed at 100°C for 10 minutes. Next, to take the second step of phosphonium compound formation, 315 µmol (50 eq. relative to precursor) of the corresponding bromide (alkylating agent) was added to the vial, and a phosphonium compound formation reaction was performed at 100°C for an appropriate time for each case. After the reaction, the vial was lifted from the oil bath, and 5 mL of water was added to the vial. The solution contained in the vial was introduced into a Sep-Pak® tC18 cartridge which had been activated in advance by introducing 5 mL of ethanol and water (10 mL), and then the Sep-Pak® tC18 cartridge was washed by introducing water (10 mL). After air was introduced to remove water in the cartridge, the solution containing an [¹⁸F]-labeled compound was eluted from the cartridge using ethanol (5 mL). The eluate was diluted with water (1 mL) and the resulting solution was introduced into a preparative HPLC column to effect separation and purification (preparative column: Inertsil® ODS-4, 10 mm × 250 mm; mobile phase: PBS:MeCN = 52:48; flow rate: 5.0 mL/min.; detection UV wavelength: 254 nm). The desired radioactive fraction was fractionated and diluted with pure water (20 mL). The fraction solution was introduced into a Sep-Pak® tC18 cartridge which had been activated in advance by introducing ethanol (5 mL) and water (10 mL), and then the Sep-Pak® tC18 cartridge was washed by introducing water (10 mL). After air was introduced to remove water in the cartridge, the [¹⁸F]-labeled compound was eluted from the cartridge into a vial using ethanol (5 mL). The eluate was azeotropically dried under stream of helium in an oil bath at 100°C. Thereafter, an appropriate amount of physiological saline was added to the reaction vial, and the resulting solution was used as a chemical solution for use in biological analysis.

The compounds of working examples as shown below were synthesized by the same procedure as mentioned above.

### Example 1: Benzyl-[4-(2-[¹⁸F]fluoroethoxy)phenyl]-diphenylphosphonium bromide ([¹⁸F]TAP-001)

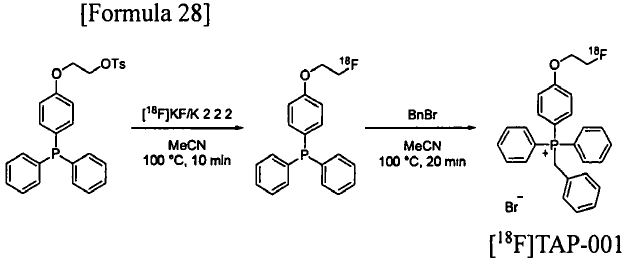

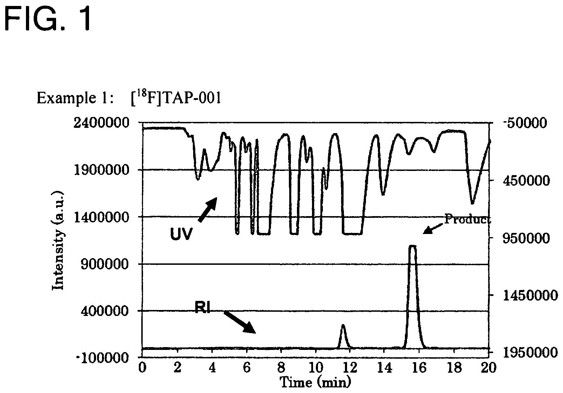

The total label synthesis time taken until the completion of preparing the formulation was about 1.5 hours, the radiochemical yield was 43±18% (corrected for attenuation), and the radiochemical purity was at least 98±1.8%. FIG. 1 shows the HPLC results obtained during purification.
HPLC conditions: column: Inertsil ODS-4 (4.6x 150 mm, particle size 5 µm); mobile phase: MeCN/PBS = 50/50; UV wavelength: 254 nm; flow rate: 1.5 mL/min.
Retention time: 4.8 min.

### Example 2: Benzyl-[2-(2-[¹⁸F]fluoroethoxy)phenyl]-diphenylphosphonium bromide ([¹⁸F]T AP-028)

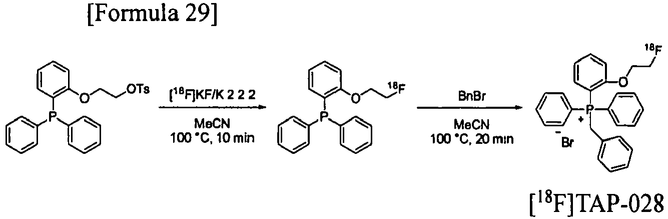

The total label synthesis time taken until the completion of preparing the formulation was about 1.5 hours, the radiochemical yield was 12±9.9% (corrected for attenuation), and the radiochemical purity was at least 92±5.8%.

HPLC conditions: column: InertSustain C-18 (4.6×150 mm, particle size 5 µm); mobile phase: MeCN/PBS = 50/50; UV wavelength: 254 nm; flow rate: 1.5 mL/min.

Retention time: 4.4 min.

### Example 3: Benzyl-[3-(2-[¹⁸ F]fluoroethoxy)phenyl]-diphenylphosphonium bromide ([¹⁸F]TAP-003)

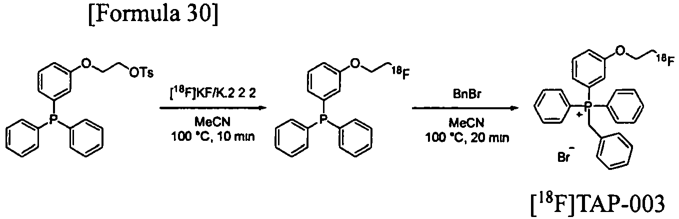

The total label synthesis time taken until the completion of preparing the formulation was about 1.5 hours, the radiochemical yield was 36±8.4% (corrected for attenuation), and the radiochemical purity was at least 99±0.1%.

HPLC conditions: column: InertSustain C-18 (4.6×150 mm, particle size 5 µm); mobile phase: MeCN/PBS = 50/50; UV wavelength: 254 nm; flow rate: 1.5 mL/min.

Retention time: 4.4 min.

### Example 4: [3-(2-[¹⁸F]fluoroethoxy)phenyl]-(4-methoxycarbonylphenyl)methyl-diphenylphosphonium bromide ([¹⁸F]TAP-020)

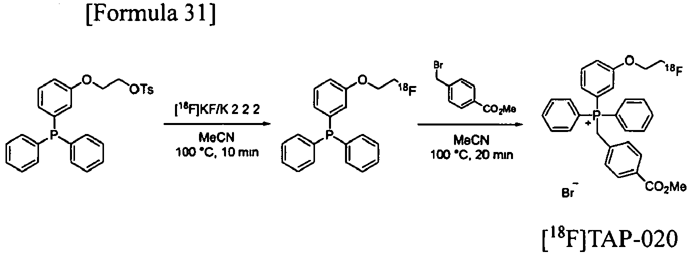

In the process of the synthesis of the labeled compound [¹⁸F]TAP-004, 4-(bromomethyl)benzoic acid methyl ester used as a reagent for phosphonium compound formation is solid; therefore, after the first step of fluorination reaction, acetonitrile was once distilled away and then a solution of the reagent in acetonitrile (72 mg (50 eq.)/600 µL) was added again, and thereafter the second reaction step was performed. The other synthesis operations were done by the same procedure as the aforementioned procedure for ¹⁸F-labeling reaction. The total label synthesis time taken until the completion of preparing the formulation was about 1.5 hours, the radiochemical yield was 32% (corrected for attenuation), and the radiochemical purity was at least 95%.

HPLC conditions: column: InertSustain C-18 (4.6×150 mm, particle size 5 µm); mobile phase: MeCN/PBS = 50/50; UV wavelength: 254 nm; flow rate: 1.0 mL/min.

Retention time: 5.5 min.

### Example 5: [3-(2-[¹⁸F]fluoroethoxy)phenyl]-(4-methoxyphenyl)methyl-diphenylphosphonium bromide ([¹⁸F]TAP-019)

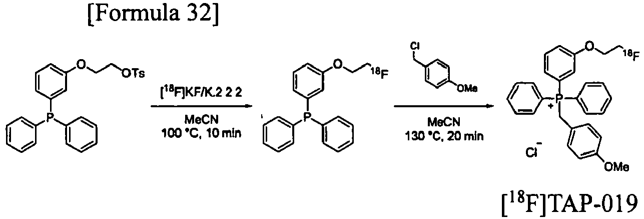

The synthesis operations were performed by the same procedure as the aforementioned procedure for ¹⁸F-labeling reaction, except that (4-methoxyphenyl)methylchloride was used as an alkylating agent and the reaction temperature was set to 130°C. The total label synthesis time taken until the completion of preparing the formulation was about 1.5 hours, the radiochemical yield was 42% (corrected for attenuation), and the radiochemical purity was at least 99%.

HPLC conditions: column: InertSustain C-18 (4.6×150 mm, particle size 5 µm); mobile phase: MeCN/PBS = 50/50; UV wavelength: 254 nm; flow rate: 1.0 mL/min.

Retention time: 6.8 min.

### Example 6: [¹⁸F][3-(2-fluoroethoxy)phenyl]-(3-phenylpropyl)-diphenylphosphonium bromide ([¹⁸F]TAP-022)

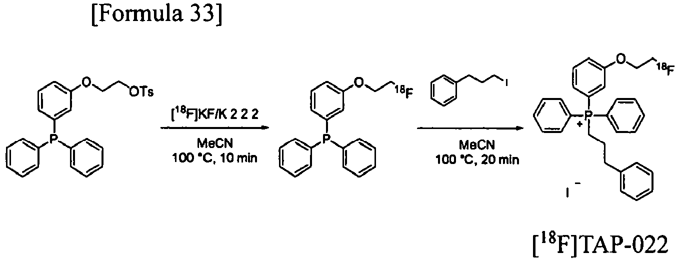

In the process of the synthesis of the labeled compound [¹⁸F]TAP-006, 3-iodopropylbenzene was used as an alkylating agent. Since 3-iodopropylbenzene is solid, after the first step of fluorination reaction, acetonitrile was once distilled away and then a solution of the reagent in acetonitrile (78 mg (50 eq.)/600 µL) was added again, and thereafter the second reaction step was performed. The other synthesis operations were done by the same procedure as the aforementioned procedure for ¹⁸F-labeling reaction. The total label synthesis time taken until the completion of preparing the formulation was about 1.5 hours, the radiochemical yield was 21% (corrected for attenuation), and the radiochemical purity was at least 99%.

HPLC conditions: column: InertSustain C-18 (4.6×150 mm, particle size 5 µm); mobile phase: MeCN/PBS = 50/50; UV wavelength: 254 nm; flow rate: 1.5 mL/min.

Retention time: 6.8 min.

Likewise, the compounds shown below were also synthesized according to the same synthesis procedure.

**[Table 3]**

| Compound | Structural formula | Electrophile | Radiochemical yield (%)/ HPLC retention time (HPLC conditions) |
|---|---|---|---|
| [¹⁸F]TAP-002 | 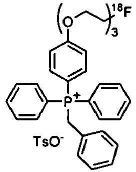 | 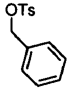 | 12% |
| | | | 5.8 min. |
| | | | (column: Inertsil ODS-4 (4.6×150 mm, particle size: 5 µm); mobile phase: MeCN/PBS = 50/50, UV wavelength: 254 nm; flow rate: 1.5 mL/min.) |
| [¹⁸F]TAP-004 | 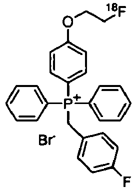 | 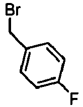 | 49% |
| | | | 6.1 min. |
| | | | (column: Inertsil ODS-4 (4.6×150 mm, particle size: 5 µm); mobile phase: MeCN/PBS = 50/50; UV wavelength: 254 nm; flow rate: 1.5 mL/min.) |
| [¹⁸F]TAP-008 | 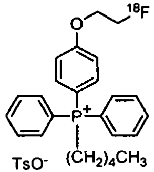 | 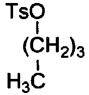 | 22% |
| | | | 4.9 min. |
| | | | (column: InertSustain C-18 (4.6×150 mm, particle size: 5 µm); mobile phase: MeCN/PBS = 50/50; UV wavelength: 254 nm; flow rate: 1.5 mL/min.) |
| [¹⁸F]TAP-024 | 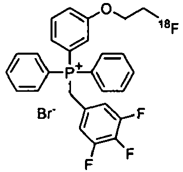 | 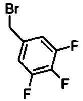 | 31% |
| | | | 5.2 min. |
| | | | (column: InertSustain C-18 (4.6×150 mm, particle size: 5 µm); mobile phase: MeCN/PBS = 50/50; UV wavelength: 254 nm; flow rate: 1.5 mL/min.) |

### Example 16: Analysis of phosphonium compound formation reaction time

The reaction according to the scheme shown below was performed by following the aforementioned procedure for ¹⁸F-labeling reaction. In this process, the reaction was performed using varied times of phosphonium compound formation reaction, whereby the resulting radiochemical yields for the different cases were compared. The results are shown in the table given below. Among the cases where the reaction time at the second step was set to 20, 10 or 5 min., the highest radiochemical yield was obtained for the case of setting the reaction time to 5 min.

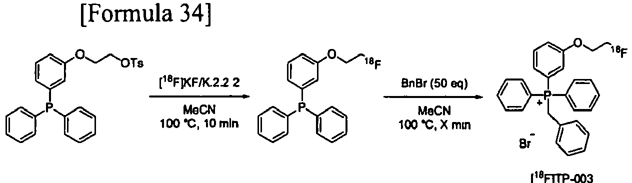

| Reaction time at 2nd step (X) | Radiochemical yield (%) |
|---|---|
| 20 min (n=4) | 36±8.4 |
| 10 min (n=3) | 39±9.3 |
| 5 min (n=13) | 47±7.4 |

### Test Example 1: Mouse PET/CT imaging

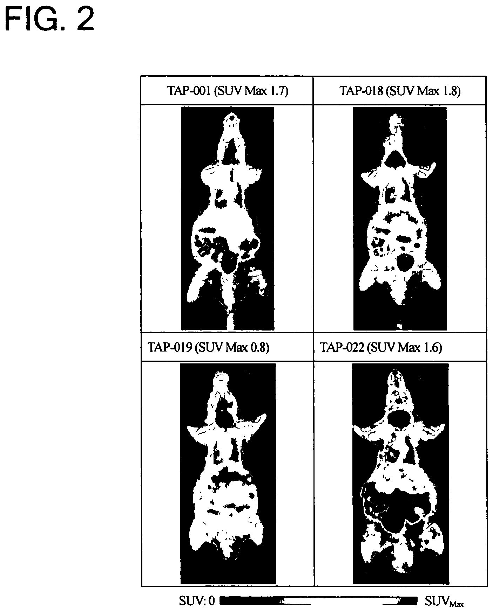

The animals used in this measurement were 6-10-week-old male Slc:ICR mice (from Japan SLC, Inc.). Each of different physiological saline solutions of the [¹⁸F]-labeled compounds (200 µL, 1.94-14.7 MBq) used in this measurement was administered via the caudal vein to the awake mice, and the mice were left to settle for 50 minutes. Then, the mice were fixed under isoflurane anesthesia (2%, 1.5 mL/min.), and after 60 minutes of the administration, the whole bodies of the mice were imaged by small-animal PET (ClairvivoPET produced by Shimadzu Corporation) for 10 minutes. Subsequently, while the mice were kept fixed, their whole bodies were imaged by small-animal CT (ClairvivoCT produced by Shimadzu Corporation). The PET data were reconstructed by 3D-DRAMA, and the pixel values of the obtained PET images were converted to SUV to generate SUV images. These images were fused with CT images to produce fused images. The results are shown in FIG. 2.

The accumulation of radioactivity in the myocardium was observed in all the cases of using [¹⁸F]TAP-001, -018, -019, and -022 -- this confirmed that these compounds are usable for myocardial imaging. In particular, high-contrast myocardial images were obtained in the cases of using [¹⁸F]TAP-018, -019, and -022.

### Test Example 2: Mouse biodistribution study

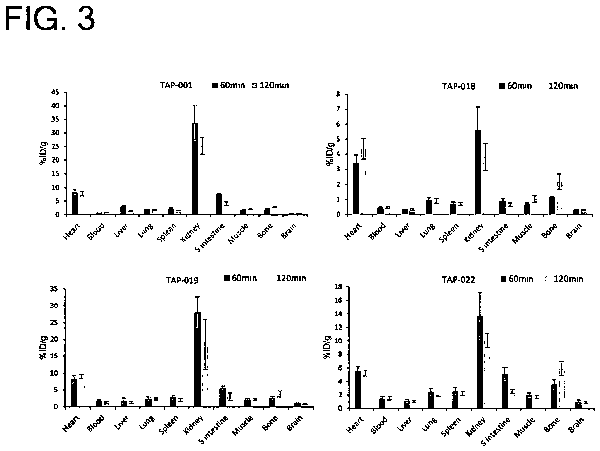

The animals used in this measurement were 6-8-week-old male Slc:ICR mice (from Japan SLC, Inc.) (n=4). Each of different physiological saline solutions of the [¹⁸F]-labeled compounds (200 µL, 370-740 kBq) used in this measurement was administered to the awake mice via the caudal vein, and after 60 or 120 minutes of the administration, the mice were euthanized by cervical dislocation under isoflurane anesthesia. After blood was sampled from the heart immediately, the heart, lung, liver, spleen, kidney, small intestine, thigh muscle, thigh bone, and brain were excised and measured for amount of radioactivity by a γ counter. The weights of the excised organs were measured, and on that basis, the accumulation rates per gram of the organs with respect to dose (% dose/g of organ,%ID/g) were calculated as indicators for accumulation of the chemical compounds used (n=4). The results are shown in FIG. 3.

The accumulation of labeled compound in the myocardium was observed in all the cases of using [¹⁸F]TAP-001, -018, -019, and -022 -- this confirmed that the radioactivity more highly accumulates in the myocardium than in other adjacent organs. In particular, the ratio of radioactivity accumulations in the myocardium and liver (heart/liver ratio) -- those accumulations were considered most likely to affect imaging -- was calculated and found to be 2.70 for [¹⁸F]TAP-001, 9.40 for [¹⁸F]TAP-018, 4.56 for [¹⁸F]TAP-0 19, or 4.98 for [¹⁸F]TAP-022, at the time point of 60 minutes. This demonstrated that all the compounds accumulate in a heart-specific manner.

### Test Example 3: Rat biodistribution study

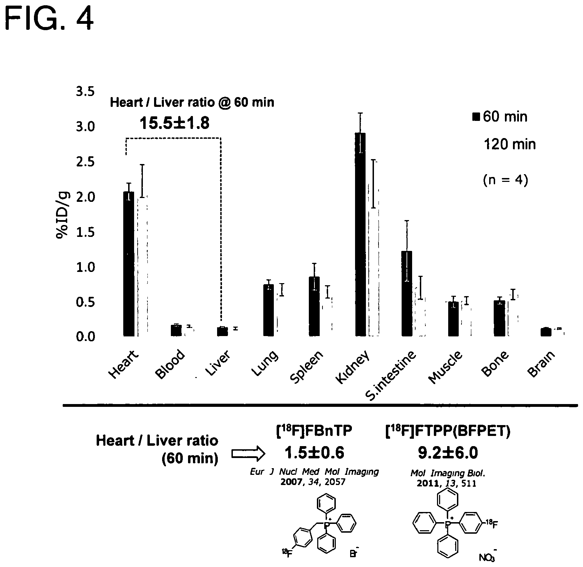

The biodistribution study of [¹⁸F]TAP-018 in rats was conducted by the same procedure as in Test Example 2 using 6-week-old male Wistar rats (from Japan SLC, Inc.) (n=4). The results are summarized in a bar graph (FIG. 4). [¹⁸F]TAP-018 highly accumulated in the heart, and the heart/liver ratio was found to be about 15.5 at the time point of 60 minutes. This value is about ten times higher than the value of 1.5 for [¹⁸F]FBnTP which has been used as a gold standard, and is sufficiently higher than the value of 9.2 for [¹⁸F]FTPP which is currently under clinical study. This demonstrates that [¹⁸F]TAP-018 is useful as a myocardial perfusion imaging agent.

### Test Example 4: Small-animal PET study in rats

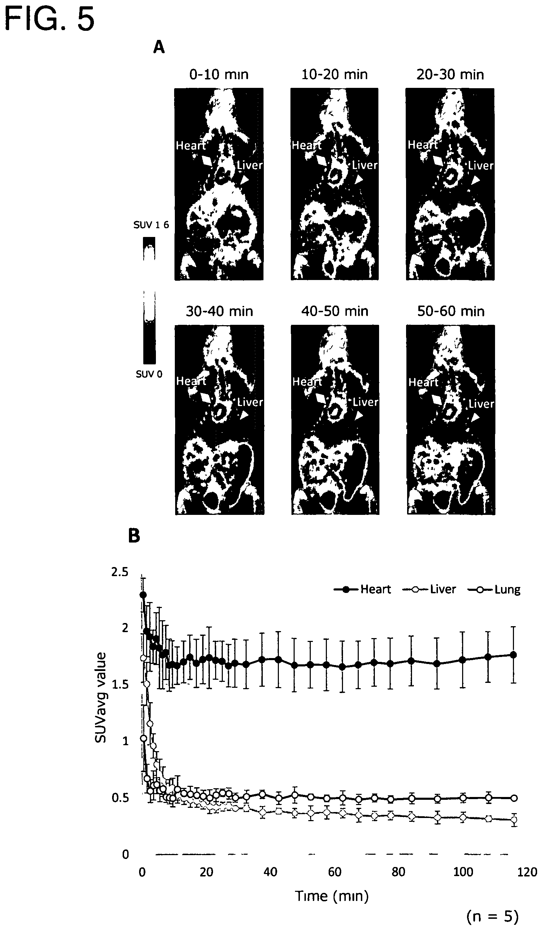

The myocardial imaging study of [¹⁸F]TAP-018 in rats was conducted using 6-week-old male Wistar rats. 200 µL (7.83-9.66 MBq) of a physiological saline solution of the test compound [¹⁸F]TAP-018 was administered via the caudal vein to the rats fixed under isoflurane anesthesia (2%, 1.5 mL/min.), and then the whole bodies of the rats were imaged by small-animal PET at time points from 0 to 120 minutes after the administration. Subsequently, while the rats were kept fixed, their whole bodies were imaged by small-animal CT. The PET data were reconstructed by 3D-DRAMA, and the pixel values of the obtained PET images were converted to SUV to generate SUV images. These images were fused with CT images to produce fused images. Further, the locations of the myocardium, lung and liver were identified based on the fused images, and regions of interest (ROI) were drawn to calculate respective SUVavg values. FIG. 5A shows the PET images taken until 60 minutes after the administration. FIG. 5B shows the radioactivity-time curves in the heart, liver and lung. As evident, radioactivity disappeared rapidly from the liver until 10 minutes after the administration, whereas a high level of radioactivity accumulated in the heart immediately after the administration. These results of PET imaging demonstrated that [¹⁸F]TAP-018 is useful as a myocardial perfusion agent.

## Claims

1. A phosphonium compound of formula (II): 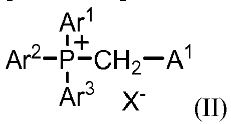
wherein Ar¹, Ar², and Ar³ are each independently an aryl optionally substituted with one or more substituents selected from B², wherein at least one of Ar¹, Ar², and Ar³ is substituted with one or more substituents selected from radionuclide-labeled C₁₋₆alkyl, radionuclide-labeled C₂₋₆alkoxy, radionuclide-labeled C₂₋₆alkoxyC₁₋₆alkyl, radionuclide-labeled C₂₋₆alkoxyC₂₋₆alkoxy, radionuclide-labeled C₂₋₆alkoxyC₂₋₆alkoxyC₁₋₆alkyl, and radionuclide-labeled C₂₋₆alkoxyC₂₋₆alkoxyC₂₋₆alkoxy;
A¹ is a hydrogen atom, C₁₋₁₀alkyl optionally substituted with one or more substituents selected from B¹, C₂₋₁₀alkenyl optionally substituted with one or more substituents selected from B¹, or an aryl optionally substituted with one or more substituents selected from B²;
each B¹ is independently a halogen atom, C₁₋₆alkoxy, phenyl, or naphthyl, wherein the phenyl and naphthyl are optionally substituted with one or more substituents selected from C₁₋₆alkyl, C₁₋₆alkoxy, and halogen atoms;
each B² is independently a halogen atom, C₁₋₆alkyl, C₁₋₆alkoxy, C₁₋₆alkylthio, cyano, amino, C₁₋₆alkylamino, di(C₁₋₆alkyl)amino, nitro, hydroxy, or (C₁₋₃alkoxy)carbonyl, wherein the alkyl, alkoxy and alkylthio are optionally substituted with one or more substituents selected from halogen atoms;
Ar¹, Ar², Ar³ and A¹, and the substituents contained therein, optionally form acid addition salts;
X⁻ is an anion with a total charge of -1.

2. The phosphonium compound according to claim 1, wherein the radionuclide is a positron emitting nuclide, preferably ¹⁸F.

3. The phosphonium compound according to claim 1 or 2, wherein A¹ is a hydrogen atom, C₁₋₆alkyl optionally substituted with one or more halogen atoms, phenyl, or phenylC₁₋₄alkyl, wherein the phenyl or a phenyl moiety of phenylC₁₋₄alkyl is optionally substituted with one or more substituents selected from one or more halogen atoms, C₁₋₆alkyl, C₁₋₆alkoxy, C₁₋₆alkylthio, cyano, amino, C₁₋₆alkylamino, di(C₁₋₆alkyl)amino, nitro, hydroxy, and (C₁₋₃alkoxy)carbonyl.

4. The phosphonium compound according to claim 1, wherein the phosphonium compound comprises a phosphonium selected from:
benzyl-[4-(2- [¹⁸F]fluoroethoxy)phenyl]-diphenylphosphonium;
benzyl-(4-[2-{2-(2-[¹⁸F]fluoroethoxy)ethoxy}ethoxy]phenyl)-diphenylphosphonium;
[4-(2-[¹⁸F]fluoroethoxy)phenyl]-(4-methoxycarbonylphenyl)methyl-diphenylphosphonium;
[4-(2-[¹⁸F]fluoroethoxy)phenyl]-(4-fluorophenyl)methyl-diphenylphosphonium;
[4-(2-[¹⁸F]fluoroethoxy)phenyl]-(3,4,5-trifluorophenyl)methyl-diphenylphosphonium;
(4-chlorophenyl)methyl-[4-(2-[¹⁸F]fluoroethoxy)phenyl]-diphenylphosphonium;
[4-(2-[¹⁸F]fluoroethoxy)phenyl]-(4-methoxyphenyl)methyl-diphenylphosphonium;
[4-(2-[¹⁸F]fluoroethoxy)phenyl]-(n-pentyl)-diphenylphosphonium;
[4-(2-[¹⁸F]fluoroethoxy)phenyl]-(3-phenylpropyl)-diphenylphosphonium;
(4-n-butylphenyl)methyl-[4-(2-[¹⁸F]fluoroethoxy)phenyl]-diphenylphosphonium;
(3-fluorophenyl)methyl-[4-(2-[¹⁸F]fluoroethoxy)phenyl]-diphenylphosphonium;
[4-(2-[¹⁸F]fluoroethoxy)phenyl]-[4-(trifluoromethylthio)phenyl]methyl-diphenylphosphonium;
[4-(2-[¹⁸F]fluoroethoxy)phenyl]-(2-methylphenyl)methyl-diphenylphosphonium;
(3-cyanophenyl)methyl-[4-(2-[¹⁸F]fluoroethoxy)phenyl]-diphenylphosphonium;
[ 4-(2-[¹⁸F]fluoroethoxy)phenyl]-methyl-diphenylphosphonium;
allyl-[4-(2-[¹⁸F]fluoroethoxy)phenyl]-diphenylphosphonium;
benzyl-[3-(2-[¹⁸F]fluoroethoxy)phenyl]-diphenylphosphonium;
[3-(2-[¹⁸F]fluoroethoxy)phenyl]-(4-methoxyphenyl)methyl-diphenylphosphonium;
[3-(2-[¹⁸F]fluoroethoxy)phenyl]-(4-methoxycarbonylphenyl)methyl-diphenylphosphonium;
[3-(2-[¹⁸F]fluoroethoxy)phenyl]-(4-fluorophenyl)methyl-diphenylphosphonium;
[3-(2-[¹⁸F]fluoroethoxy)phenyl]-(3-phenylpropyl)-diphenylphosphonium;
(4-chlorophenyl)methyl-[3-(2-[¹⁸F]fluoroethoxy)phenyl]-diphenylphosphonium;
[3-(2-[¹⁸F]fluoroethoxy)phenyl]-(3,4,5-trifluorophenyl)methyl-diphenylphosphonium;
benzyl-[3-(3-[^{1g}F]fluoropropoxy)phenyl]-diphenylphosphonium;
benzyl-[3-(4-[¹⁸F]fluorobutoxy)phenyl]-diphenylphosphonium;
[3-(2-[¹⁸F]fluoroethoxy)phenyl]-methyl-diphenylphosphonium; and
benzyl-[2-(2-[¹⁸F]fluoroethoxy)phenyl]-diphenylphosphonium.

5. The phosphonium compound according to claim 1, wherein the phosphonium compound is selected from:
benzyl-[4-(2-[¹⁸F]fluoroethoxy)phenyl]-diphenylphosphonium bromide;
benzyl-[3-(2-[¹⁸F]fluoroethoxy)phenyl]-diphenylphosphonium bromide;
[3-(2-[¹⁸F]fluoroethoxy)phenyl]-(4-methoxyphenyl)methyl-diphenylphosphonium bromide; and
[3-(2-[¹⁸F]fluoroethoxy)phenyl]-(3-phenylpropyl)-diphenylphosphonium bromide.

6. A radiopharmaceutical for use in imaging, comprising the phosphonium compound according to any one of claims 1 to 5.

7. A radiopharmaceutical for use in PET imaging, comprising the phosphonium compound according to any one of claims 2 to 5.

8. The radiopharmaceutical according to claim 7, for use in imaging of myocardium, tumor, or brown adipose tissue.

9. A method for producing a quaternary phosphonium compound labeled with a radionuclide of Formula (II) according to claim 1, the method comprising the step of:
reacting an electrophile of formula (I): X¹-CH₂-A¹ with triphenylphosphine having one or more radionuclide-labeled substituents on the benzene ring to give a quaternary phosphonium salt;
wherein X¹ is a leaving group;
A¹ is a hydrogen atom, C₁₋₁₀alkyl optionally substituted with one or more substituents selected from B¹, C₂₋₁₀alkenyl optionally substituted with one or more substituents selected from B¹, or an aryl optionally substituted with one or more substituents selected from B²;
each B¹ is independently a halogen atom, C₁₋₆alkoxy, phenyl, or naphthyl, wherein the phenyl and naphthyl are optionally substituted with one or more substituents selected from C₁₋₆alkyl, C₁₋₆alkoxy, and halogen atoms;
each B² is independently a halogen atom, C₁₋₆alkyl, C₁₋₆alkoxy, C₁₋₆alkylthio, cyano, amino, C₁₋₆alkylamino, di(C₁₋₆alkyl)amino, nitro, hydroxy, or (C₁₋₃alkoxy)carbonyl, wherein the alkyl, alkoxy and alkylthio are optionally substituted with one or more substituents selected from halogen atoms;
wherein the one or more radionuclide-labeled substituents are selected from radionuclide-labeled C₁₋₆alkyl, radionuclide-labeled C₂₋₆alkoxy, radionuclide-labeled C₂₋₆alkoxyC₁₋₆alkyl, radionuclide-labeled C₂₋₆alkoxyC₂₋₆alkoxy, radionuclide-labeled C₂₋₆alkoxyC₂₋₆alkoxyC₁₋₆alkyl, and radionuclide-labeled C₂₋₆alkoxyC₂₋₆alkoxyC₂₋₆alkoxy;
wherein the triphenylphosphine optionally further has one or more substituents selected from B² on the benzene ring.

10. The method according to claim 9, wherein the radionuclide is a positron emitting nuclide, preferably ¹⁸F.

11. The method according to claim 9 or 10, wherein X¹ is a halogen atom, optionally substituted C₁₋₆alkylsulfonyloxy, or optionally substituted phenylsulfonyloxy.

12. The method according to any one of claims 9 to 11, wherein the method is performed in an automatic synthesizer.

13. Use of a phosphonium compound as a reference standard for a labeled compound according to any one of claims 1 to 5, wherein the phosphonium compound corresponds to the labeled compound defined in any one of claims 1 to 5 except that the radionuclide of the labeled compound is replaced with a non-radioactive identical element.

14. Use of a phosphine compound of formula (III) for producing the phosphonium compound according to any one of claims 1 to 5: 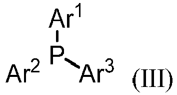
wherein Ar¹, Ar², and Ar³ are each independently an aryl optionally substituted with one or more substituents selected from B²;
wherein at least one of Ar¹, Ar², and Ar³ is substituted with one or more substituents selected from: C₁₋₆alkyl optionally substituted with one or more substituents selected from L, C₂₋₆alkoxy optionally substituted with one or more substituents selected from L, C₂₋₆alkoxyC₁₋₆alkyl optionally substituted with one or more substituents selected from L, C₂₋₆alkoxyC₂₋₆alkoxy optionally substituted with one or more substituents selected from L, C₂₋₆alkoxyC₂₋₆alkoxyC₁₋₆alkyl optionally substituted with one or more substituents selected from L, and C₂₋₆alkoxyC₂₋₆alkoxyC₂₋₆alkoxy optionally substituted with one or more substituents selected from L;
B² is a halogen atom, C₁₋₆alkyl, C₁₋₆alkoxy, C₁₋₆alkylthio, cyano, amino, C₁₋₆alkylamino, di(C₁₋₆alkyl)amino, nitro, hydroxy, or (C₁₋₃alkoxy)carbonyl, wherein the alkyl, alkoxy, and alkylthio are optionally substituted with one or more substituents selected from halogen atoms;
L is bromine, iodine, p-toluenesulfonyloxy, methanesulfonyloxy, chloromethanesulfonyloxy, or trifluoromethanesulfonyloxy.

15. The use according to claim 14, wherein the phosphine compounds is comprised in a kit.

## Patentansprüche

1. Eine Phosphoniumverbindung der Formel (II): 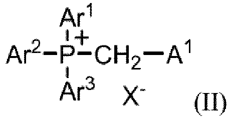
wobei Ar¹, Ar² und Ar³ jeweils unabhängig ein Aryl darstellen, gegebenenfalls substituiert mit einem oder mehreren Substituierten, ausgewählt aus B², wobei mindestens eines von Ar¹, Ar² und Ar³ mit einem oder mehreren Substituenten, ausgewählt aus mit Radionuklid-markiertem C₁₋₆Alkyl, mit Radionuklid-markiertem C₂₋₆Alkoxy, mit Radionuklid-markiertem C₂₋₆AlkoxyC₁₋₆alkyl, mit Radionuklid-markiertem C₂₋₆AlkoxyC₂₋₆alkoxy, mit Radionuklid-markiertem C₂₋₆Alkoxy-C₂₋₆alkoxyC₁₋₆alkyl und mit Radionuklid-markiertem C₂₋₆AlkoxyC₂₋₆alkoxyC₂₋₆alkoxy, substituiert sind;
A¹ ein Wasserstoffatom, C₁₋₁₀Alkyl gegebenenfalls substituiert mit einem oder mehreren Substituenten, ausgewählt aus B¹, C₂₋₁₀Alkenyl gegebenenfalls substituiert mit einem oder mehreren Substituenten, ausgewählt aus B¹, oder ein Aryl gegebenenfalls substituiert mit einem oder mehreren Substituenten, ausgewählt aus B², darstellt;
jedes B¹ unabhängig ein Halogenatom, C₁₋₆Alkoxy, Phenyl oder Naphthyl darstellt, wobei das Phenyl und Naphthyl gegebenenfalls mit einem oder mehreren Substituenten, ausgewählt aus C₁₋₆Alkyl, C₁₋₆Alkoxy und Halogenatomen substituiert sind;
jedes B² unabhängig ein Halogenatom, C₁₋₆Alkyl, C₁₋₆Alkoxy, C₁₋₆Alkylthio, Cyano, Amino, C₁₋₆Alkylamino, Di(C₁₋₆alkyl)amino, Nitro, Hydroxy oder (C₁₋₃Alkoxy)carbonyl darstellt, wobei das Alkyl, Alkoxy und Alkylthio gegebenenfalls mit einem oder mehreren Substituenten, ausgewählt aus Halogenatomen, substituiert sind;
Ar¹, Ar², Ar³ und A¹ und die darin enthaltenen Substituenten gegebenenfalls Säureadditionssalze bilden;
X⁻ ein Anion mit einer Gesamtladung von -1 darstellt.

2. Die Phosphoniumverbindung gemäß Anspruch 1, wobei das Radionuklid ein Positron emittierendes Nuklid ist, vorzugsweise ¹⁸F.

3. Die Phosphoniumverbindung gemäß Anspruch 1 oder 2, wobei A¹ ein Wasserstoffatom, C₁₋₆Alkyl gegebenenfalls substituiert mit einem oder mehreren Halogenatomen, Phenyl oder PhenylC₁₋₄alkyl ist, wobei das Phenyl oder eine Phenyleinheit von PhenylC₁₋₄alkyl gegebenenfalls substituiert mit einem oder mehreren Substituenten, ausgewählt aus einem oder mehreren Halogenatomen, C₁₋₆Alkyl, C₁₋₆Alkoxy, C₁₋₆Alkylthio, Cyano, Amino, C₁₋₆Alkylamino, Di(C₁₋₆alkyl)amino, Nitro, Hydroxy und (C₁₋₃Alkoxy)carbonyl, ist.

4. Die Phosphoniumverbindung gemäß Anspruch 1, wobei die Phosphoniumverbindung ein Phosphonium umfasst, welches ausgewählt ist aus:
Benzyl-[4-(2-[¹⁸F]fluorethoxy)phenyl]-diphenylphosphonium;
Benzyl-(4-[2-{2-(2-[¹⁸F]fluorethoxy)ethoxy}ethoxy]phenyl)-diphenylphosphonium;
[4-(2-[¹⁸F]Fluorethoxy)phenyl]-(4-methoxycarbonylphenyl)methyl-diphenyl-phosphonium;
[4-(2-[¹⁸F]Fluorethoxy)phenyl]-(4-fluorphenyl)methyl-diphenylphosphonium;
[4-(2-[¹⁸F]Fluorethoxy)phenyl]-(3,4,5-trifluorphenyl)methyl-diphenylphosphonium;
(4-Chlorphenyl)methyl-[4-(2-[¹⁸F]fluorethoxy)phenyl]-diphenylphosphonium;
[4-(2-[¹⁸F]Fluorethoxy)phenyl]-(4-methoxyphenyl)methyl-diphenylphosphonium;
[4-(2-[¹⁸F]Fluorethoxy)phenyl]-(n-pentyl)-diphenylphosphonium;
[4-(2-[¹⁸F]Fluorethoxy)phenyl]-(3-phenylpropyl)-diphenylphosphonium;
(4-n-Butylphenyl)methyl-[4-(2-[¹⁸F]fluorethoxy)phenyl]-diphenylphosphonium;
(3-Fluorphenyl)methyl-[4-(2-[¹⁸F]fluorethoxy)phenyl]-diphenylphosphonium;
[4-(2-[¹⁸F]Fluorethoxy)phenyl]-[4-(trifluormethylthio)phenyl]methyl-diphenylphosphonium;
[4-(2-[¹⁸F]Fluorethoxy)phenyl]-(2-methylphenyl)methyl-diphenylphosphonium;
(3-Cyanophenyl)methyl-[4-(2-[¹⁸F]fluorethoxy)phenyl]-diphenylphosphonium;
[4-(2-[¹⁸F]Fluorethoxy)phenyl]methyl-diphenylphosphonium;
Allyl-[4-(2-[¹⁸F]Fluorethoxy)phenyl]-diphenylphosphonium;
Benzyl-[3-(2-[¹⁸F]fluorethoxy)phenyl]-diphenylphosphonium;
[3-(2-[¹⁸F]Fluorethoxy)phenyl]-(4-methoxyphenyl)methyl-diphenylphosphonium;
[3-(2-[¹⁸F]Fluorethoxy)phenyl]-(4-methoxycarbonylphenyl)methyldiphenyl-phosphonium;
[3-(2-[¹⁸F]Fluorethoxy)phenyl]-(4-fluorphenyl)methyl-diphenylphosphonium;
[3-(2-[¹⁸F]Fluorethoxy)phenyl]-(3-phenylpropyl)-diphenylphosphonium;
(4-Chlorphenyl)methyl-[3-(2-[¹⁸F]fluorethoxy)phenyl]-diphenylphosphonium;
[3-(2-[¹⁸F]Fluorethoxy)phenyl]-(3,4,5-trifluorphenyl)methyl-diphenylphosphonium;
Benzyl-[3-(3-[¹⁸F]fluorpropoxy)phenyl]-diphenylphosphonium;
Benzyl-[3-(4-[¹⁸F]fluorbutoxy)phenyl]-diphenylphosphonium;
[3-(2-[¹⁸F]Fluorethoxy)phenyl]-methyl-diphenylphosphonium; und
Benzyl-[2-(2-[¹⁸F]fluorethoxy)phenyl]-diphenylphosphonium.

5. Die Phosphoniumverbindung gemäß Anspruch 1, wobei die Phosphoniumverbindung ausgewählt ist aus:
Benzyl-[4-(2-[¹⁸F]fluorethoxy)phenyl]-diphenylphosphoniumbromid;
Benzyl-[3-(2-[¹⁸F]fluorethoxy)phenyl]-diphenylphosphoniumbromid;
[3-(2-[¹⁸F]Fluorethoxy)phenyl]-(4-methoxyphenyl)methyl-diphenylphosphonium-bromid; und
[3-(2-[¹⁸F]Fluorethoxy)phenyl]-(3-phenylpropyl)-diphenylphosphoniumbromid.

6. Ein Radiopharmakon zur Verwendung bei der Bildgebung, umfassend die Phosphoniumverbindung gemäß einem der Ansprüche 1 bis 5.

7. Ein Radiopharmakon zur Verwendung bei der PET-Bildgebung, umfassend die Phosphoniumverbindung gemäß einem der Ansprüche 2 bis 5.

8. Das Radiopharmakon gemäß Anspruch 7 zur Verwendung bei der Bildgebung von Myokard-, Tumor- oder braunem Fettgewebe.

9. Ein Verfahren zur Herstellung einer quaternären Phosphoniumverbindung, welche mit einem Radionuklid der Formel (II) gemäß Anspruch 1 markiert ist, wobei das Verfahren den Schritt umfasst:
Umsetzen eines Elektrophils der Formel (I): X¹-CH₂-A¹ mit Triphenylphosphin, welches einen oder mehrere mit Radionuklid-markierte(n) Substituenten an dem Benzolring aufweist, um ein quaternäres Phosphoniumsalz zu erhalten;
wobei X¹ eine Abgangsgruppe ist;
A¹ ein Wasserstoffatom, C₁₋₁₀Alkyl gegebenenfalls substituiert mit einem oder mehreren Substituenten, ausgewählt aus B¹, C₂₋₁₀Alkenyl gegebenenfalls substituiert mit einem oder mehreren Substituenten, ausgewählt aus B¹, oder ein Aryl gegebenenfalls substituiert mit einem oder mehreren Substituenten, ausgewählt aus B², darstellt;
jedes B¹ unabhängig ein Halogenatom, C₁₋₆Alkoxy, Phenyl oder Naphthyl darstellt, wobei das Phenyl und Naphthyl gegebenenfalls mit einem oder mehreren Substituenten, ausgewählt aus C₁₋₆Alkyl, C₁₋₆Alkoxy und Halogenatomen substituiert sind;
jedes B² unabhängig ein Halogenatom, C₁₋₆Alkyl, C₁₋₆Alkoxy, C₁₋₆Alkylthio, Cyano, Amino, C₁₋₆Alkylamino, Di(C₁₋₆alkyl)amino, Nitro, Hydroxy oder (C₁₋₃Alkoxy)carbonyl darstellt, wobei das Alkyl, Alkoxy und Alkylthio gegebenenfalls mit einem oder mehreren Substituenten, ausgewählt aus Halogenatomen, substituiert sind;
wobei der eine oder die mehreren mit Radionuklid-markierten Substituenten ausgewählt sind aus mit Radionuklid-markiertem C₁₋₆Alkyl, mit Radionuklid-markiertem C₂₋₆Alkoxy, mit Radionuklid-markiertem C₂₋₆AlkoxyC₁₋₆alkyl, mit Radionuklid-markiertem C₂₋₆AlkoxyC₂₋₆alkoxy, mit Radionuklid-markiertem C₂₋₆Alkoxy-C₂₋₆alkoxyC₁₋₆alkyl und mit Radionuklid-markiertem C₂₋₆AlkoxyC₂₋₆alkoxyC₂₋₆alkoxy, substituiert sind;
wobei das Triphenylphosphin gegebenenfalls ferner einen oder mehrere Substituenten, ausgewählt aus B², an dem Benzolring aufweist.

10. Das Verfahren gemäß Anspruch 9, wobei das Radionuklid ein Positron emittierendes Nuklid ist, vorzugsweise ¹⁸F.

11. Das Verfahren gemäß Anspruch 9 oder 10, wobei X¹ ein Halogenatom, gegebenenfalls substituiertes C₁₋₆Alkylsulfonyloxy oder gegebenenfalls substituiertes Phenylsulfonyloxy ist.

12. Das Verfahren gemäß einem der Ansprüche 9 bis 11, wobei das Verfahren in einem automatischen Synthetisator durchgeführt wird.

13. Verwendung einer Phosphoniumverbindung als ein Referenzstandard für eine markierte Verbindung gemäß einem der Ansprüche 1 bis 5, wobei die Phosphoniumverbindung der markierten Verbindung wie in einem der Ansprüche 1 bis 5 definiert entspricht, mit der Ausnahme, dass das Radionuklid der markierten Verbindung mit einem nichtradioaktiven identischen Element ersetzt wird.

14. Verwendung einer Phosphinverbindung der Formel (III) zur Herstellung der Phosphoniumverbindung gemäß einem der Ansprüche 1 bis 5: 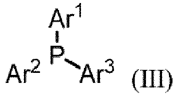
wobei Ar¹, Ar² und Ar³ jeweils unabhängig ein Aryl darstellen, welches gegebenenfalls mit einem oder mehreren Substituenten, ausgewählt aus B², substituiert sind;
wobei mindestens eines von Ar¹, Ar² und Ar³ substituiert ist mit einem oder mehreren Substituenten, ausgewählt aus: C₁₋₆Alkyl, gegebenenfalls substituiert mit einem oder mehreren Substituenten, ausgewählt aus L, C₂₋₆Alkoxy gegebenenfalls substituiert mit einem oder mehreren Substituenten, ausgewählt aus L, C₂₋₆AlkoxyC₁₋₆alkyl gegebenenfalls substituiert mit einem oder mehreren Substituenten, ausgewählt aus L, C₂₋₆AlkoxyC₂₋₆alkoxy gegebenenfalls substituiert mit einem oder mehreren Substituenten, ausgewählt aus L, C₂₋₆AlkoxyC₂₋₆alkoxyC₁₋₆alkyl gegebenenfalls substituiert mit einem oder mehreren Substituenten, ausgewählt aus L, und C₂₋₆AlkoxyC₂₋₆alkoxyC₂₋₆alkoxy gegebenenfalls substituiert mit einem oder mehreren Substituenten, ausgewählt aus L;
B² ein Halogenatom, C₁₋₆Alkyl, C₁₋₆Alkoxy, C₁₋₆Alkylthio, Cyano, Amino, C₁₋₆Alkylamino, Di(C₁₋₆alkyl)amino, Nitro, Hydroxy oder (C₁₋₃Alkoxy)carbonyl darstellt, wobei das Alkyl, Alkoxy und Alkylthio gegebenenfalls mit einem oder mehreren Substituenten, ausgewählt aus Halogenatomen, substituiert sind;
L Brom, Iod, p-Toluolsulfonyloxy, Methansulfonyloxy, Chlormethansulfonyloxy oder Trifluormethansulfonyloxy darstellt.

15. Die Verwendung gemäß Anspruch 14, wobei die Phosphinverbindung in einem Kit umfasst ist.

## Revendications

1. Composé de phosphonium de formule (II) : 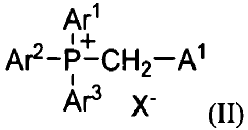
dans laquelle chacun de Ar¹, Ar² et Ar³ est indépendamment un aryle éventuellement substitué par un ou plusieurs substituants choisis parmi B², et au moins l'un parmi Ar¹, Ar² et Ar³ est substitué par un ou plusieurs substituants choisis parmi alkyle en C₁ à C₆ marqué par un radionucléide, alcoxy en C₂ à C₆ marqué par un radionucléide, (alcoxy en C₂ à C₆)-alkyle en C₁ à C₆ marqué par un radionucléide, (alcoxy en C₂ à C₆)-alcoxy en C₂ à C₆ marqué par un radionucléide, (alcoxy en C₂ à C₆)-(alcoxy en C₂ à C₆)-alkyle en C₁ à C₆ marqué par un radionucléide, et (alcoxy en C₂ à C₆)-(alcoxy en C₂ à C₆)-alcoxy en C₂ à C₆ marqué par un radionucléide ;
A¹ est un atome d'hydrogène, alkyle en C₁ à C₁₀ éventuellement substitué par un ou plusieurs substituants choisis parmi B¹, alcényle en C₂ à C₁₀ éventuellement substitué par un ou plusieurs substituants choisis parmi B¹, ou aryle éventuellement substitué par un ou plusieurs substituants choisis parmi B² ;
chaque B¹ est indépendamment un atome d'halogène, alcoxy en C₁ à C₆, phényle ou naphtyle, lesquels phényle et naphtyle sont éventuellement substitués par un ou plusieurs substituants choisis parmi alkyle en C₁ à C₆, alcoxy en C₁ à C₆ et les atomes d'halogène ;
chaque B² est indépendamment un atome d'halogène, alkyle en C₁ à C₆, alcoxy en C₁ à C₆, alkylthio en C₁ à C₆, cyano, amino, alkylamino en C₁ à C₆, di(alkyle en C₁ à C₆)amino, nitro, hydroxy, ou (alcoxy en C₁ à C₃)carbonyle, lesquels alkyle, alcoxy et alkylthio sont éventuellement substitués par un ou plusieurs substituants choisis parmi les atomes d'halogène ;
Ar¹, Ar², Ar³ et A¹, et les substituants qu'ils contiennent, forment éventuellement des sels d'addition d'acide ;
X⁻ est un anion ayant une charge totale de -1.

2. Composé de phosphonium selon la revendication 1, dans lequel le radionucléide est un nucléide émettant des positons, de préférence ¹⁸F.

3. Composé de phosphonium selon la revendication 1 ou 2, dans lequel A¹ est un atome d'hydrogène, alkyle en C₁ à C₆ éventuellement substitué par un ou plusieurs atomes d'halogène, phényle, ou phényl-alkyle en C₁ à C₄, lequel phényle ou fragment phényle du phényl-alkyle en C₁ à C₄ est éventuellement substitué par un ou plusieurs substituants choisis parmi un ou plusieurs atomes d'halogène, alkyle en C₁ à C₆, alcoxy en C₁ à C₆, alkylthio en C₁ à C₆, cyano, amino, alkylamino en C₁ à C₆, di(alkyle en C₁ à C₆)amino, nitro, hydroxy, et (alcoxy en C₁ à C₃)carbonyle.

4. Composé de phosphonium selon la revendication 1, lequel composé de phosphonium comprend un phosphonium choisi parmi les suivants :
benzyl-[4-(2-[¹⁸F]fluoroéthoxy)phényl]-diphénylphosphonium ;
benzyl-(4-[2-{2-(2-[¹⁸F]fluoroéthoxy)éthoxy}éthoxy]phényl)-diphénylphosphonium ;
[4-(2-[¹⁸F]fluoroéthoxy)phényl]-(4-méthoxycarbonylphényl)méthyl-diphénylphosphonium ;
[4-(2-[¹⁸F]fluoroéthoxy)phényl]-(4-fluorophényl)méthyl-diphénylphosphonium ;
[4-(2-[¹⁸F]fluoroéthoxy)phényl]-(3,4,5-trifluorophényl)méthyl-diphénylphosphonium ;
(4-chlorophényl)méthyl-[4-(2-[¹⁸F]fluoroéthoxy)phényl]-diphénylphosphonium ;
[4-(2-[¹⁸F]fluoroéthoxy)phényl]-(4-méthoxyphényl)méthyl-diphénylphosphonium ;
[4-(2-[¹⁸F]fluoroéthoxy)phényl]-(n-pentyl)-diphénylphosphonium ;
[4-(2-[¹⁸F]fluoroéthoxy)phényl]-(3-phénylpropyl)-diphénylphosphonium ;
(4-n-butylphényl)méthyl-[4-(2-[¹⁸F]fluoroéthoxy)phényl]-diphénylphosphonium ;
(3-fluorophényl)méthyl-[4-(2-[¹⁸F]fluoroéthoxy)phényl]-diphénylphosphonium ;
[4-(2-[¹⁸F]fluoroéthoxy)phényl]-[4-(trifluorométhylthio)phényl]méthyl-diphénylphosphonium ;
[4-(2-[¹⁸F]fluoroéthoxy)phényl]-(2-méthylphényl)méthyl-diphénylphosphonium ;
(3-cyanophényl)méthyl-[4-(2-[¹⁸F]fluoroéthoxy)phényl]-diphénylphosphonium ;
[4-(2-[¹⁸F]fluoroéthoxy)phényl]-méthyl-diphénylphosphonium ;
allyl-[4-(2-[¹⁸F]fluoroéthoxy)phényl]-diphénylphosphonium ;
benzyl-[3-(2-[¹⁸F]fluoroéthoxy)phényl]-diphénylphosphonium ;
[3-(2-[¹⁸F]fluoroéthoxy)phényl]-(4-méthoxyphényl)méthyl-diphénylphosphonium ;
[3-(2-[¹⁸F]fluoroéthoxy)phényl]-(4-méthoxycarbonylphényl)méthyl-diphénylphosphonium ;
[3-(2-[¹⁸F]fluoroéthoxy)phényl]-(4-fluorophényl)méthyl-diphénylphosphonium ;
[3-(2-[¹⁸F]fluoroéthoxy)phényl]-(3-phénylpropyl)-diphénylphosphonium ;
(4-chlorophényl)méthyl-[3-(2-[¹⁸F]fluoroéthoxy)phényl]-diphénylphosphonium ;
[3-(2-[¹⁸F]fluoroéthoxy)phényl]-(3,4,5-trifluorophényl)méthyl-diphénylphosphonium ;
benzyl-[3-(3-[¹⁸F]fluoropropoxy)phényl]-diphénylphosphonium ;
benzyl-[3-(4-[¹⁸F]fluorobutoxy)phényl]-diphénylphosphonium ;
[3-(2-[¹⁸F]fluoroéthoxy)phényl]-méthyl-diphénylphosphonium ; et
benzyl-[2-(2-[¹⁸F]fluoroéthoxy)phényl]-diphénylphosphonium.

5. Composé de phosphonium selon la revendication 1, lequel composé de phosphonium est choisi parmi les suivants :
bromure de benzyl-[4-(2-[¹⁸F]fluoroéthoxy)phényl]-diphénylphosphonium ;
bromure de benzyl-[3-(2-[¹⁸F]fluoroéthoxy)phényl]-diphénylphosphonium ;
bromure de [3-(2-[¹⁸F]fluoroéthoxy)phényl]-(4-méthoxyphényl)méthyl-diphénylphosphonium ; et
bromure de [3-(2-[¹⁸F]fluoroéthoxy)phényl]-(3-phénylpropyl)-diphénylphosphonium.

6. Radiopharmaceutique pour une utilisation en imagerie, comprenant le composé de phosphonium de l'une quelconque des revendications 1 à 5.

7. Radiopharmaceutique pour une utilisation en imagerie PET, comprenant le composé de phosphonium de l'une quelconque des revendications 2 à 5.

8. Radiopharmaceutique selon la revendication 7, pour une utilisation en imagerie du myocarde, d'une tumeur, ou d'un tissu adipeux brun.

9. Procédé pour produire un composé de phosphonium quaternaire marqué par un radionucléide de formule (II) selon la revendication 1, le procédé comprenant les étapes suivantes :
réaction d'un électrophile de formule (I) : X¹-CH₂-A¹ avec de la triphénylphosphine ayant un ou plusieurs substituants marqués par un radionucléide sur le cycle benzène, ce qui donne un sel de phosphonium quaternaire ;
où X¹ est un groupe partant ;
A¹ est un atome d'hydrogène, alkyle en C₁ à G₁₀ éventuellement substitué par un ou plusieurs substituants choisis parmi B¹, alcényle en C₂ à C₁₀ éventuellement substitué par un ou plusieurs substituants choisis parmi B¹, ou aryle éventuellement substitué par un ou plusieurs substituants choisis parmi B² ;
chaque B¹ est indépendamment un atome d'halogène, alcoxy en C₁ à C₆, phényle ou naphtyle, lesquels phényle et naphtyle sont éventuellement substitués par un ou plusieurs substituants choisis parmi alkyle en C₁ à C₆, alcoxy en C₁ à C₆ et les atomes d'halogène ;
chaque B² est indépendamment un atome d'halogène, alkyle en C₁ à C₆, alcoxy en C₁ à C₆, alkylthio en C₁ à C₆, cyano, amino, alkylamino en C₁ à C₆, di(alkyle en C₁ à C₆)amino, nitro, hydroxy, ou (alcoxy en C₁ à C₃)carbonyle, lesquels alkyle, alcoxy et alkylthio sont éventuellement substitués par un ou plusieurs substituants choisis parmi les atomes d'halogène ;
dans lequel le ou les substituants marqués par un radionucléide sont choisis parmi alkyle en C₁ à C₆ marqué par un radionucléide, alcoxy en C₂ à C₆ marqué par un radionucléide, (alcoxy en C₂ à C₆)-alkyle en C₁ à C₆ marqué par un radionucléide, (alcoxy en C₂ à C₆)-alcoxy en C₂ à C₆ marqué par un radionucléide, (alcoxy en C₂ à C₆)-(alcoxy en C₂ à C₆)-alkyle en C₁ à C₆ marqué par un radionucléide, et (alcoxy en C₂ à C₆)-(alcoxy en C₂ à C₆)-alcoxy en C₂ à C₆ marqué par un radionucléide ;
dans lequel la triphénylphosphine a éventuellement en outre un ou plusieurs substituants choisis parmi B² sur le cycle benzène.

10. Procédé selon la revendication 9, dans lequel le radionucléide est un nucléide émettant des positons, de préférence ¹⁸F.

11. Procédé selon la revendication 9 ou 10, dans lequel X¹ est un atome d'halogène, alkylsulfonyloxy en C₁ à C₆ éventuellement substitué, ou phénylsulfonyloxy éventuellement substitué.

12. Procédé selon l'une quelconque des revendications 9 à 11, lequel procédé est mis en œuvre dans un synthétiseur automatique.

13. Utilisation d'un composé de phosphonium en tant qu'étalon de référence pour un composé marqué selon l'une quelconque des revendications 1 à 5, dans laquelle le composé de phosphonium correspond au composé marqué défini dans l'une quelconque des revendications 1 à 5, sauf que le radionucléide du composé marqué est remplacé par un élément identique non radioactif.

14. Utilisation d'un composé de phosphine de formule (III) pour produire le composé de phosphonium de l'une quelconque des revendications 1 à 5 : 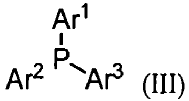
dans laquelle chacun de Ar¹, Ar² et Ar³ est indépendamment un aryle éventuellement substitué par un ou plusieurs substituants choisis parmi B² ;
dans laquelle au moins l'un parmi Ar¹, Ar² et Ar³ est substitué par un ou plusieurs substituants choisis parmi : alkyle en C₁ à C₆ éventuellement substitué par un ou plusieurs substituants choisis parmi L, alcoxy en C₂ à C₆ éventuellement substitué par un ou plusieurs substituants choisis parmi L, (alcoxy en C₂ à C₆)-alkyle en C₁ à C₆ éventuellement substitué par un ou plusieurs substituants choisis parmi L, (alcoxy en C₂ à C₆)-alcoxy en C₂ à C₆ éventuellement substitué par un ou plusieurs substituants choisis parmi L, (alcoxy en C₂ à C₆)-(alcoxy en C₂ à C₆)-alkyle en C₁ à C₆ éventuellement substitué par un ou plusieurs substituants choisis parmi L, et (alcoxy en C₂ à C₆)-(alcoxy en C₂ à C₆)-alcoxy en C₂ à C₆ éventuellement substitué par un ou plusieurs substituants choisis parmi L ;
B² est un atome d'halogène, alkyle en C₁ à C₆, alcoxy en C₁ à C₆, alkylthio en C₁ à C₆, cyano, amino, alkylamino en C₁ à C₆, di(alkyle en C₁ à C₆)amino, nitro, hydroxy, ou (alcoxy en C₁ à C₃)carbonyle, lesquels alkyle, alcoxy et alkylthio sont éventuellement substitués par un ou plusieurs substituants choisis parmi les atomes d'halogène ;
L est le brome, l'iode, p-toluènesulfonyloxy, méthanesulfonyloxy, chlorométhanesulfonyloxy, ou trifluorométhanesulfonyloxy.

15. Utilisation selon la revendication 14, dans laquelle le composé de phosphine est compris dans une trousse.
